# EUROPEAN PATENT APPLICATION

(11) **EP 4 851 628 A1**
(43) Date of publication of application: **09.12.2026**
(21) Application number: 25749056.5
(22) Date of filing: 31.01.2025
(51) Int. Cl.: G01N 33/543, G16H 10/40

(54) **SYSTEM AND METHOD FOR SUPPORTING BLOOD GLUCOSE MANAGEMENT OF SUBJECT USING COMPUTER SYSTEM**

(30) Priority: 01.02.2024 JP 2024014322
(71) Applicant: Provigate Inc., Tokyo 113-0023 (JP)
(72) Inventor: MINOURA Itsushi, Tokyo 113-0033 (JP)
(74) Representative: Michalski Hüttermann & Partner mbB
(86) International application number: PCT/JP2025/003223
(87) International publication number: WO 2025/164770

(57) **Abstract**

According to an embodiment of the present disclosure, a method for supporting glycemic management of a subject using a computer system is provided. The computer system includes a processor and a communication unit connected to the processor and capable of communicating with an electronic terminal that can be accessed by the subject. The method includes: acquiring, by the processor via the communication unit, GP[i] and GP[j], which are target protein glycation ratios (GP levels) of the subject at two times t[i] and t[j] > t[i]; calculating, by the processor using GP[i] and GP[j] as a basis, a glucose management index (GMI[i, j]) that is a mean blood glucose level equivalent to GP levels in a period between the two times t[i] and t[j]; and generating, by the processor, output information based on the glucose management index (GMI[i, j]) and outputting, by the processor via the communication unit, the output information to the subject.

## Description

### TECHNICAL FIELD

The present disclosure relates to a technology for supporting blood glucose management of a subject using a computer system.

### BACKGROUND ART

Diabetes is an endocrine disorder characterized by chronically high blood glucose levels (such as high fasting blood glucose levels and/or high HbA1c levels). Diabetes is mainly classified into type 1 diabetes, type 2 diabetes, gestational diabetes, diabetes caused by pathological variants of related genes, diabetes associated with other diseases, and other types. Type 2 diabetes, which accounts for the majority of cases, is mainly caused by decreased insulin secretion or by insulin resistance. Type 1 diabetes is caused by the destruction of beta cells in the pancreas.

Regardless of type, the basic treatment involves drug therapy in addition to diet therapy and exercise therapy. These treatments can provide appropriate blood glucose management and reduce the risk of complications. In other words, lifestyle improvements are important. Furthermore, there are patients referred to as "prediabetic" or "borderline diabetes" who do not meet the diagnostic criteria for diabetes, but have high blood glucose levels. For patients of this type, lifestyle improvements are important to reduce the risk of developing diabetes. As above, lifestyle improvements, including appropriate diet and exercise, are thought to be effective in blood glucose management and prevention of diabetes.

The treatment of diabetes, a chronic disease, is generally considered difficult. Some of the reasons for this include: the disease progresses without subjective symptoms; maintaining motivation for treatment is difficult; complete cure is difficult and treatment must be continued throughout life; and not only drug therapy but also lifestyle improvements are essential. These are considered to make the self-management necessary for treatment difficult (NPL 1).

In fact, it has been reported that it takes between 18 and 254 days, with a median of 66 days, for a new behvaior to become automatic (NPL 2). Based on this, behavior change requires two to several months. In general, some kind of system is needed to maintain motivation for such a long period of time.

Typical examples of biomarkers for glycemic control are blood glucose levels, HbA1c levels, and GA levels. However, fasting blood glucose levels can hardly be considered to reflect lifestyle behaviors. HbA1c is considered to reflect blood glucose levels over the past two to three months. However, it is difficult to maintain the motivation for self-management over such a long period. In other words, HbA1c levels are too long-term to serve as feedback for behavior change.

Recently, large amounts of data obtained from continuous glucose monitoring (CGM) are being used. CGM is a technology for continuously measuring glucose levels in interstitial fluid at 15-minute intervals for approximately 14 days. This technology helps visualize changes in blood glucose levels, including blood glucose trends, postprandial hyperglycemia and other blood glucose spikes, and nocturnal hypoglycemia. However, it has been pointed out that absolute values are not necessarily accurate, and the large amounts of data obtained are difficult to handle for the patients themselves and for physicians and other healthcare professionals.

On the other hand, GA levels represent the degree of glycation of the protein albumin. Since GA levels are relative values indicating the amount of glycated albumin with respect to the amount of total albumin, techniques have been developed to measure GA levels with high accuracy. Also, albumin has a blood half-life of about 17 days, which is close to the human behavioral cycle of one week. By checking their own GA levels weekly, subjects are more likely to maintain motivation for making lifestyle improvements on their own. Accordingly, GA levels have been receiving much attention in recent years as a marker for glycemic management.

Nevertheless, the half-life of albumin is 17 days, which is longer than one week. For this reason, a subject's behavior changes from week to week are not immediately represented as the weekly GA levels. To evaluate a certain behavior change in terms of GA levels, it is necessary to observe GA levels over several weeks.

### CITATION LIST

### NON-PATENT LITERATURE

NPL 1: American Diabetes Association, Diagnosis and classification of diabetes mellitus, Diabetes Care. 2014; 37 Suppl 1:S81-90. doi: 10.2337/dc14-S081., https://doi.org/10.2337/dc14-s081
NPL 2: Lally P, van Jaarsveld C, Potts H, Wardle J. How are habits formed: Modelling habit formation in the real world. Eur J Soc Psychol. 2009; 40(6):998-1009. doi:10.1002/ejsp.674

### SUMMARY OF INVENTION

Although GA levels are excellent glycemic management markers, users want to know immediately how their efforts for the week are reflected in the weekly GA levels. Meeting this demand is important to maintain the motivation of the person in question. The method of the present disclosure was arrived at as a result of intensive efforts by the inventor to solve this problem.

According to some embodiments of the present disclosure, there is provided calculating, using GA[i] and GA[j] obtained at two times t[i] and t[j], respectively, as a basis, a mean blood glucose level equivalent to the GA levels in that period as a GA glucose management index (GMI_{GA}[i, j]). In some embodiments, there is provided performing, using this GA glucose management index (GMI_{GA}[i, j]), glucose management, glycemic management, lifestyle management, and the like.

In some embodiments, a goal of a subject is acquired. In some embodiments, a GA level of the subject is acquired. In some embodiments, GA levels of the subject at two times are acquired. In some embodiments, the two GA levels are used as a basis to calculate a GA glucose management index (GMI_{GA}[i, j]) as the mean blood glucose level equivalent to the GA levels in that period. In some embodiments, the calculated glucose management index is outputted to the subject.

In some embodiments, the glucose management index GMI is more generally defined as the mean blood glucose level calculated by converting from a protein glycation ratio. For example, the GMI may be used as an HbA1c GMI value (GMI_{HbA1c}).

In an embodiment, the present disclosure may also cause a computer system to execute the method. In some embodiments, the computer system may be provided with: a processor; a communication unit connected to the processor and capable of communicating with a user interface (for example, a display unit and/or an electronic terminal that can be accessed by the subject); and a memory connected to the processor. In some embodiments, the computer is made to acquire GA levels (GA[i], GA[j]) of the subject at two times (t[i], t[j]). In some embodiments, the computer is made to use the acquired two GA levels (GA[i], GA[j]) as a basis to calculate a glucose management index (GMI[i, j]) as the mean blood glucose level equivalent to the GA levels in that period. In some embodiments, the computer is made to output the calculated glucose management index to a terminal of the subject.

### ADVANTAGEOUS EFFECTS OF INVENTION

The general life cycle in modern times is established using the week as a unit of time. Consequently, users can more directly perceive how their actual behavior in a given week affected their GA levels, which are physiological markers. Consequently, for example, the present invention is effective in raising users' awareness of their lifestyle behaviors, inducing users to make behavior changes on their own, and maintaining users' motivation.

Note that the above effects are not necessarily limiting, and any effect indicated in this specification or another effect that may be understood from this specification may also be exhibited together with, or instead of, the above effects.

### BRIEF DESCRIPTION OF DRAWINGS

Fig. 1 illustrates a flowchart of a method according to an embodiment.
Fig. 2 illustrates a flowchart of a method according to an embodiment.
Fig. 3A illustrates a flowchart of a method according to an embodiment.
Fig. 3B illustrates a flowchart of a method according to an embodiment.
Fig. 3C illustrates a flowchart of a method according to an embodiment.
Fig. 3D illustrates a flowchart of a method according to an embodiment.
Fig. 4A illustrates a network configuration including a system and a subject terminal according to an embodiment.
Fig. 4B illustrates a block diagram representing functional components of a system according to an embodiment.
Fig. 5A illustrates graphs representing changes in GA levels and a WGMI according to an example.
Fig. 5B illustrates a graph representing changes in GA levels and a WGMI according to an example.
Fig. 5C illustrates a graph representing changes in GA levels and a WGMI according to an example.
Fig. 5D illustrates graphs representing changes in GA levels, a WGMI, and HbA1c levels according to an example.
Fig. 6 illustrates a graph representing a correlation between CGM and a GMI according to an example.
Fig. 7A illustrates a graph illustrating a correlation between predicted and actual levels when HbA1c levels are predicted using a conventional method.
Fig. 7B illustrates a graph illustrating a correlation between predicted and actual levels when HbA1c levels are predicted using a method according to an example of the present disclosure.
Fig. 7C illustrates a graph illustrating a correlation between predicted and actual levels when HbA1c levels are predicted using a method according to another example of the present disclosure.
Fig. 8 illustrates a graph (a) representing change over time in GA levels, GMI_{GA}, and HbA1c levels actually obtained in a clinical study according to an example, and a graph (b) representing change over time in HbA1c levels predicted according to two methods.
Fig. 9 illustrates a scatter plot (a) illustrating the relation between R_{GI} and BMI for each participant obtained from clinical study data, a scatter plot (b) illustrating the relation between R_{GI} and waist circumference of each participant, and a scatter plot (c) representing the relation between R_{GI} and HDL cholesterol level of each participant.

### DESCRIPTION OF EMBODIMENTS

In the following description, numerous specific details are given to provide a more complete understanding of the present invention. However, it will be apparent to a person skilled in the art that the present invention can be practiced in one or more of these specific details. In other examples, features and procedures well known to a person skilled in the art are not described to avoid obscuring the present invention.

Some embodiments and examples are described with reference to the attached drawings. Arrows in the sequence diagrams indicate the order of information transfer or processes (steps). On the other hand, no order is specified between processes not specified by arrows, unless there is a contradiction. The nature of these arrows applies to other sequence diagrams. Note that when multiple embodiments and drawings have similar functions, configurations or processes, the similar functions, configurations, or processes may be omitted from one of the embodiments and drawings. Such omissions are made on the assumption that the functions, configurations, or processes may be understood by a person skilled in the art. This does not necessarily mean that the functions, configurations, or processes are the same in the multiple embodiments and drawings.

Unless otherwise defined, all technical and scientific terms used in this specification have the same meaning as generally understood by a person skilled in the art of the technical field to which the present invention belongs.

The term "subject" used in this specification refers to the entity that performs, or should perform, glycemic management. The term "user" used in this specification refers to the entity that receives output from the system. In some embodiments, the user may be the subject themselves who performs glycemic management. In some embodiments, the user may be a person with a lawful right to perform glycemic management of the subject independently or in cooperation with the subject, such as a healthcare professional or a family member of the subject. The user may also encompass multiple entities. "Subject" or "user" encompasses any subject who is currently using, should use in the future, is planning to use, or has used the system according to the present disclosure.

In some embodiments, the subject may be a human being. However, the subject according to the present disclosure is not limited to a human being. Examples of the subject include animals other than human beings. For example, the subject may be a mammal. Examples of the subject include, but are not limited to, competition livestock (such as racehorses), working animals, domestic animals, pets, and wild animals.

The subject may be a patient with diabetes. For example, the patient with diabetes may be a patient with type 2 diabetes. The subject may be a non-diabetic patient. A non-diabetic patient refers to all human beings who have not been diagnosed with diabetes. The subject may be pre-diabetic and/or healthy.

### Computer

The calculation of the GMI from acquired GA levels, the prediction of future (unmeasured) GA levels and/or HbA1c levels based on the GMI value, the output of information, optimization, and other calculation steps in the present disclosure are performed by a computer or a computer unit (hereinafter also referred to as a "computational unit") unless otherwise specified.

### System

In some embodiments, the system may be provided with a computational unit (for example, a processor). The system may be configured to include a stationary server or a computational unit. The system may be configured to include a user interface (for example, a display unit and/or a stationary or mobile electronic terminal used by the user, for example). The system may be provided with hardware and software for executing a method according to the present disclosure. For example, the system may include an application installed in a mobile electronic terminal in the possession of the user.

In some embodiments, the system may be provided with a computational unit (for example, a processor) and a sensor. The sensor may be configured to measure a body fluid sample from the user and calculate a glycoalbumin (GA) level. Specifically, for example, the sensor may be configured to measure the total albumin amount and the glycated albumin amount in a body fluid sample, or may be configured to obtain the ratio of the glycated albumin amount to the total albumin amount in a body fluid sample directly, without obtaining the individual amounts.

In some embodiments, the system may be provided with: a computational unit (for example, a processor); a sensor; a communication unit connected to the processor and capable of communicating with a user interface (for example, a display unit and/or an electronic terminal that can be accessed by the subject); and a memory connected to the processor.

In the present disclosure, when the entity of an action is not specified, the entity is to be interpreted as being the system.

The system may be configured to allow access to GA levels of the subject, input information from the subject, and output information to the subject only to the subject or another person with a legitimate right, and to block access to such information by other persons without a legitimate right. In an embodiment or example according to the present disclosure, the input and output of information between a single subject and the system are mainly described, but the present invention is not limited thereto. A method, system, or the like according to the present disclosure may also be configured such that services can be provided to multiple or numerous subjects. Input information from numerous subjects, processing and optimization of the information, secondary data processing, output information to numerous subjects, and the like may be referenced to execute information processing and optimization, secondary data processing, generation of output information, and the like with respect to each subject.

### GA level measurement

The term "GA level" used in this specification is generally an amount calculated by dividing the amount of glycoalbumin in body fluid of the subject by the total amount of albumin in the same body fluid. Alternatively, the GA level may be obtained as the ratio of the glycated albumin amount to the total albumin amount in a body fluid sample, without obtaining the individual amounts.

Examples of methods for measuring GA levels include, but are not limited to, enzymatic methods, high-performance liquid chromatography (HPLC), mass spectrometry, and isotope dilution mass spectrometry. An enzyme sensor, an HPLC apparatus, a mass spectrometer (MS apparatus), or the like can be used as a device or sensor to measure GA levels.

In some embodiments, enzymatic methods may be used as clinical testing methods. Glycoalbumin may be broken down into amino acids by proteases, ketoamine oxidase, which reacts only with glycated amino acids, may be applied, and the resulting H₂O₂ generated thereby may be measured to obtain the glycoalbumin concentration. Albumin concentration may also be calculated by reacting with bromocresol purple (BCP) reagent and measuring the change in absorbance of the blue conjugate.

For example, the GA level can be calculated as [glycoalbumin concentration / albumin concentration] × 100(%). Alternatively, a relational formula and conversion formula (conversion from mmol/mol to %) between the analysis value of a glycoalbumin standard specimen and a GA level linked to the standard specimen may be prepared in advance. The relational formula may be found by linear regression of the two values. The GA level can be calculated on the basis of the relational formula by measuring the total albumin concentration and the glycoalbumin concentration in a specimen.

In some embodiments, a glycoprotein sensor with immobilized protease, immobilized ketoamine oxidase, and a hydrogen peroxide detection part may be used to measure the concentration of glycoalbumin.

For example, such a sensor can be used for minimally invasive measurement of GA levels from not only venous blood, but also from minute amounts of blood such as fingertip blood, earlobe blood, heel blood, capillary blood, dried blood spot (DBS), and skin puncture blood. Alternatively, the concentration of glycoalbumin in tear fluid or saliva can be measured non-invasively. Consequently, GA levels can be measured without placing physical or mental stress on the examinee, or while minimizing the burden of such stress on the examinee.

### GA level acquisition

In some embodiments, the subject may acquire their own body fluid (for example, blood, saliva, and/or urine) and have the body fluid transported according to a prescribed method to a laboratory with equipment for measuring GA levels. The transportation may be by mail. The laboratory receiving the body fluid of the subject may obtain the GA level in the body fluid. In some embodiments, the laboratory may transmit the obtained GA level to a computational unit of the system. This allows the system to acquire the GA level. In some embodiments, the laboratory may save an acquired GA level in designated storage, and a processor of the system may access the same storage to acquire the GA level.

In some embodiments, the subject may personally visit a healthcare facility at a hospital, pharmacy, school, convenience store, fitness club, government office, community center, or other public facility (hereinafter also referred to simply as "hospital" or "healthcare facility"). For example, the subject may visit a healthcare facility for submission or measurement of a specimen. The healthcare facility may acquire body fluid of the subject, and then measure the GA level on-premises or at an attached hospital. The GA level measuring equipment may be, for example, point-of-care testing (POCT) equipment (hereinafter also referred to simply as "POCT equipment") or in vitro diagnostics (IVD) medical equipment. The healthcare facility may acquire body fluid of the subject and transport the body fluid to an outside laboratory and have the laboratory measure the GA level. In some embodiments, the healthcare facility may transmit the obtained GA level to a computational unit of the system. This allows the system to acquire the GA level. In some embodiments, the healthcare facility may save an acquired GA level in designated storage, and a processor of the system may access the same storage to acquire the GA level.

In some embodiments, the subject may be in possession of GA level measuring equipment at the subject's home or place of residence. The GA level measuring equipment may be, for example, POCT equipment, in vitro diagnostics (IVD) medical equipment, home medical equipment, a self-measuring instrument, or a self-monitoring apparatus. The subject may obtain body fluid on their own or have another person obtain the subject's body fluid, and then measure the GA level with the same measuring equipment. In some embodiments, the self-monitoring apparatus may transmit the calculated GA level to a computational unit of the system. This allows the system to acquire the GA level. In some embodiments, the self-monitoring apparatus may save an acquired GA level in designated storage, and a processor of the system may access the same storage to acquire the GA level.

GA levels do not necessarily have to be obtained by measurement. In some embodiments, GA levels may be set in any way, or may be calculated or estimated on the basis of another parameter such as a glucose management index.

### GA level acquisition times

The term "GA(t[i])" or "GA[i]" used in this specification means the GA level acquired at a time t[i]. Unless otherwise defined, the time t[i] means the time at which body fluid was collected for the purpose of measuring GA levels, or at least GA level measurement is one such purpose.

The expressions "time of GA level", "GA level measurement time", and "time at which GA level is/was acquired" or similar time-related expressions used in this specification are, unless otherwise defined, based not on the time at which the GA level was measured but on the time at which the body fluid or specimen containing glycoalbumin and albumin serving as the basis of the GA level was acquired.

The units of time may be "days", and more precisely may be "hours" or "hours, minutes". The time t[i] may be the day on which blood was drawn or the day on which saliva was collected, for example. In other words, GA[i] means the GA level based on body fluid collected at the time t[i] among multiple consecutive GA level measurements.

Unless otherwise defined, in this specification, GA measurements or body fluid collection for GA measurements are taken discontinuously or discretely. Consequently, expressions expressed using brackets, such as t[i] and GA[i], mean discrete time series data, where i is an integer. On the other hand, expressions expressed using parentheses, such as GA(t[i]), mean a function of which the argument is the time t or the like.

### GA level acquisition interval

For example, the frequency of GA level measurements may be substantially 1 time/month, 2 times/month, or a higher value. For example, the frequency may also be a value such as 20 times/month, 10 times/month, 8 times/month, or a lower value. For example, the frequency may be substantially 2 times/month, 3 times/month, or 4 times/month, and may also be 0.5 times/week or 1 time/week.

In some embodiments, the frequency may be substantially one time every 17 days, and in some embodiments, the frequency may be substantially around one time every 2 weeks.

GA levels may be measured at intervals averaging 7 days, averaging 10 days, averaging 14 days, or a longer interval. GA levels may be measured at intervals averaging 21 days, averaging 24 days, averaging 28 days, or a shorter interval.

The term "weekly GA level" used in this specification refers to the GA level of body fluid acquired at roughly one-week intervals. The units of the measurement interval may be set to weeks (such as Monday to Sunday, Sunday to Saturday, or Saturday to Friday). Body fluid may be collected one time at some point during the week. Consequently, the interval of GA level acquisition does not necessarily have to be fixed. In some embodiments, GA levels may be acquired once during a predetermined cycle (for example, one week). The interval, cycle, or frequency may be adjusted or varied at the subject's convenience. For example, it may not be possible to collect body fluid on the same day of the week each week, or in other words, the day of the week on which body fluid is collected may vary from week to week. For example, the cycle need not be one week. For some subjects, the unit of lifestyle is not one week. For example, a cycle such as 5 days, 6 days, 8 days, or 9 days may also be adopted. Lifestyle may also change over the course of units such as several weeks or several months. Every time lifestyle changes, the interval, cycle, or frequency setting may also be changed.

In some embodiments, the interval of actually drawing blood may be equal to or greater than 1 day and less than 14 days. In some embodiments, a plan may be formulated so that the subject's blood is drawn at an interval equal to or greater than 1 day and less than 14 days, or in other words, once a week as specified. The actual blood drawing interval does not necessarily have to be once a week as specified. The blood drawing intervals may have missing intervals or unequal intervals.

### Glucose management index (GMI)

In the present disclosure, a new glucose management index (GMI) and a glucose management method using the GMI are proposed. The glucose management index (GMI) used in this specification is expressed in terms of a mean blood glucose level equivalent to GA levels by the following equation:
[Eq. 1] ${GMI}_{GA} \left[i, j\right] = \frac{GA \left[j\right] - GA \left[i\right]}{{C}_{GA} \left[i, j\right]} + GA \left[i\right]$ (Eq. 1) where
GA[i] and GA[j] are the GA levels (% or mmol/mol) at t[i] and t[j] > t[i], respectively, ${C}_{GA} \left[i, j\right] = 1 - exp \left[-\left(t\left[j\right]-t\left[i\right]\right)/{τ}_{Alb}\right] ,$ and
τ_{Alb} is the time constant for albumin decrease (-half-life / LN(0.5); about 24.5 days).

Using GA[i-1] and GA[i], which are the GA levels at two consecutive actual or virtual measurement times t[i-1] and t[i], the glucose management index (GMI[i-1, i]) for the period from t[i-1] to t[i] is expressed by the following equation:
[Eq. 2] ${GMI}_{GA} \left[i-1,i\right] = \frac{GA \left[i\right] - GA \left[i-1\right]}{{C}_{GA} \left[i-1,i\right]} + GA \left[i\right]$ (Eq. 2)

Consequently, the units of the glucose management index (GMI) are the same as the GA levels, and are expressed in % or mmol/mol.

In this specification, the interval Δt[i] = t[i] - t[i-1] of acquiring GA levels is not limited. In some embodiments, Δt[i] = t[i] - t[i-1] may be one week. For example, Δt[i] may be equal to or greater than 1 day and less than 14 days.

In this specification, the glucose management index (GMI) based on two consecutively obtained weekly GA levels is referred to as the "weekly glucose management index (WGMI)".

### HbA1c prediction

In some embodiments, current and future HbAlc may be predicted on the basis of the GMI.

In some embodiments, current and future HbAlc may be predicted on the basis of the GMI by using the following equation:
[Eq. 3] $\begin{matrix}ha \left[i\right] = GMI \left[i-1,i\right] \times {C}_{HbA 1 c} \times a + \left(1-{C}_{HbA 1 c}\right) \times ha \left[0\right] \\ HA \left[i\right] = ha \left[i\right] + b\end{matrix}$ (Eq. 3) where
ha[i] is a corrected HbA1c level at the time t[i] (> t[0]),
HA[i] is the HbAlc level (NGSP level) at the time t[i], ${C}_{HbA 1 c} = 1 - exp \left[-\left(t\left[i\right]-t\left[0\right]\right)/{τ}_{Hb}\right] ,$
a and b are empirically determined coefficients, generally a = 0.25 and b = 2.0,
τ_{Hb} is the time constant for hemoglobin decrease (-half-life / LN(0.5)),
t[0] is the time at which the previous actual or reference value of HbAlc was measured, and
ha[0] is a corrected HbA1c level actually measured or set as a reference at the time t[0].

HbAlc levels can be converted from NGSP levels to IFCC levels using a commonly known conversion formula.

### Generalization

The present disclosure mainly uses the GMI based on GA levels, but is not limited thereto. The GMI can generally be applied to organic substances which have a half-life that can be scientifically conceptualized in a given system and which are subject to modification. Examples of target systems include, but are not limited to, naturally occurring systems such as living organisms and cells, and artificially or industrially formed systems such as in vitro and reactors. Examples of target organic substances include, but are not limited to, proteins, peptide fragments, lipids, and nucleic acids. A typical example of a modification according to the present disclosure is glycation, but the present disclosure is not limited thereto. Examples of sugars that contribute to glycation include, but are not limited to, glucose, fructose, and their derivatives. The following description uses glycation by glucose as a representative example.

In general, the glucose management index (GMI) for generalized glycoproteins (GPs) is expressed by the following equation:
[Eq. 4] ${GMI}_{GP} \left[i, j\right] = \frac{GP \left[j\right] - GP \left[i\right]}{{C}_{GP} \left[i, j\right]} + GP \left[i\right]$ (Eq. 4) where
GP[i] and GP[j] are the glycation ratios (% or mmol/mol) of the target protein at t[i] and t[j] > t[i], respectively, ${C}_{GP} \left[i, j\right] = 1 - exp \left[-\left(t\left[j\right]-t\left[i\right]\right)/{τ}_{P}\right] ,$ and
τ_{P} is the time constant for target protein decrease (-half-life / LN(0.5)).

Using GP[i-1] and GP[i], which are the glycation ratios of the target protein at two consecutive actual or virtual measurement times t[i-1] and t[i], the glucose management index (GMI[i-1, i]) for the period from t[i-1] to t[i] is expressed by the following equation:
[Eq. 5] ${GMI}_{GP} \left[i-1,i\right] = \frac{GP \left[i\right] - GP \left[i-1\right]}{{C}_{GP} \left[i-1,i\right]} + GP \left[i\right]$ (Eq. 5)

For example, the GMI can also be defined on the basis ofHbA1c levels as in the following equation:
[Eq. 6] ${GMI}_{HbA 1 c} \left[i, j\right] = \frac{HbA 1 c \left[j\right] - HbA 1 c \left[i\right]}{{C}_{HbA 1 c} \left[i, j\right]} + HbA 1 c \left[i\right]$ (Eq. 6) where
HbA1c[i] and HbA1c[j] are the HbAlc levels (% or mmol/mol) at t[i] and t[j] > t[i], respectively, ${C}_{HbA 1 c} \left[i, j\right] = 1 - exp \left[-\left(t\left[j\right]-t\left[i\right]\right)/{τ}_{Hb}\right] ,$ and
τ_{Hb} is the time constant for hemoglobin decrease (-half-life / LN(0.5); about 49.9 days).

In some embodiments, GMI(GMI_{GP1}) based on the degree of glycation (GP1) of a first protein (P1) may be used to calculate the degree of glycation (GP2) of a second protein (P2).

This relation is expressed by the following generalization of Eq. 3:
[Eq. 7] $\begin{matrix}gp 2 \left[i\right] = {GMI}_{GP 1} \left[i-1,i\right] \times {C}_{GP 2} \times a + \left(1-{C}_{GP 2}\right) \times gp 2 \left[i-1\right] \\ GP 2 \left[i\right] = gp 2 \left[i\right] + b\end{matrix}$ (Eq. 7) where
GMI_{GP1}[i-1, i] is the GP1-based GMI corresponding to the period from the time t[i-1] to the time t[i],
gp2[i] is a corrected GP2 level at the time t[i] (> t[0]),
GP2[i] is the GP2 level (NGSP level) at the time t[i], ${C}_{GP 2} = 1 - exp \left[-\left(t\left[i\right]-t\left[0\right]\right)/{τ}_{GP 2}\right] ,$
τ_{GP2} is the time constant for second protein (P2) decrease (-half-life / LN(0.5)),
a and b are empirically determined coefficients,
t[0] is the previous actual or reference value of GP2, and
gp2[0] is a corrected GP2 level actually measured or set as a reference at the time t[0].

### Output of glucose management index

In some embodiments, if a new GA level is acquired, the previous GA level may be referenced and the glucose management index (GMI) or the weekly glucose management index (WGMI) may be quickly outputted to the subject. The subject can know the latest transmitted GMI through the user interface of the electronic terminal or the like.

In some embodiments, the glucose management index may be provided so as to be accessible by the subject at a time other than the time of acquisition. Past values, the latest value, and predicted future values of the glucose management index may be provided so as to be accessible at a time freely chosen by the subject. The subject can check the GMI at any time and raise awareness of their own behavior.

### Behavior-related information

The term "behavior" used in this specification generally refers to lifestyle behaviors, activities, or actions related to what are called lifestyle-related illnesses, including diabetes. "Behavior" may affect lifestyle-related illness indicators such as blood glucose levels, HbA1c levels, GA levels, or other equivalent or related values or data (for example, CGM data).

In some embodiments, "behavior" includes exercise, diet, and/or medication. Exercise, diet, and medication all affect biomarkers such as blood glucose levels, HbA1c levels, and GA levels.

In some embodiments, information related to "behavior" (also referred to as "behavior-related information") may be entered from a user interface of an electronic terminal or the like. Examples of the format of the behavior-related information include, but are not limited to, text information, audio information, handwritten information, image information, location information, communication information, and biometrics.

In some embodiments, the behavior-related information may be reported or entered by the subject (self-reported information). In some embodiments, the behavior-related information may be biological information about the subject.

In some embodiments, the subject may enter selected answers to multiple-choice questions regarding exercise, diet, and medication. In some embodiments, the subject may enter sentences in response to open-ended questions.

In some embodiments, the behavior-related information (self-reported information or the like) may be entered in natural language. For example, the behavior-related information may be entered in the form of a journal. The subject can write down their behavior for the day in their own words, briefly or in detail. The subject may also put forward questions via the journal.

In some embodiments, the behavior-related information (self-reported information or the like) may be entered by the subject on their own. By entering the behavior-related information on their own, the subject can recognize their own behavior and modify their behavior voluntarily rather than compulsively. Or, the probability of doing so increases. Voluntary behavior change may contribute to high sustainability and effective glycemic control.

In some embodiments, the behavior-related information or self-reported information may be entered by a non-subject. For example, various information may be entered by an assistant (as non-limiting examples, a spouse, a family member, a friend, a nurse, a doctor, or a lawyer). The assistant may have an understanding of the subject's intentions. The assistant may also have a legitimate right.

In some embodiments, the behavior-related information or biological information may be acquired through a non-invasive device such as a wearable device or a bodyweight scale, through an invasive device such as a CGM, through various measurements in health checkups such as blood tests, and/or the like.

### Goals

The term "goal" used in this specification generally refers to a numerical value or state that the subject should achieve in terms of biological information and/or behavior (including goal behavior) related to lifestyle-related illnesses.

Examples of markers used as goals include, but are not limited to, GA levels, HbA1c levels, bodyweight, and BMI. The subject may set marker values that should be achieved or maintained at a prescribed time or during a prescribed period in the future. In some embodiments, the markers used as goals are GA levels. Examples of behaviors used as goals include, but are not limited to, diet, exercise, and medication. The subject may set details of diet, exercise, and/or medication to be implemented at a prescribed time or period in the future. In some embodiments, the behaviors used as goals are diet, exercise, and medication.

Examples of goals related to "exercise" include, but are not limited to, types of exercise (such as walking, swimming, strolling, stair climbing, and squats), times of day (such as after meals and early morning), and volume (such as frequency, intensity, number of steps, and time).

Examples of goals related to "diet" include, but are not limited to, foods to eat/avoid (avoid sweet drinks such as juice, stop snacking, avoid sweet snacks, eat fiber-rich foods such as vegetables, reduce intake of carbohydrates such as white rice and bread, reduce alcohol consumption); meal size, meal frequency, fat, carbohydrate, and sodium intake (glycemic index (GI)) or balance (FC balance or FCS balance), and meal order (such as eat vegetables first). Examples of goals related to "medication" include, but are not limited to, taking prescribed medications as directed and ensuring that no doses of the medication are missed.

In some embodiments, a "goal" may be proposed by the system and referred to by the subject. The subject may refer to or ignore the goal proposed by the system, and set a next goal on their own or enter the next goal into the system.

Having the subject voluntarily set their own goals rather than having existing goals set for them or choosing from a list of goal options is thought to be effective for behavior change.

A "goal" may change periodically or non-periodically. A new goal may be set voluntarily by the subject. For example, the subject may be given an opportunity to change the goal every week or whenever the latest GA level is outputted from the system. When given the opportunity, the subject may change the goal, or keep the same goal without changing.

A set "goal" or a "goal" to be proposed to the subject may be provided to the subject via a user interface (for example, a display unit, or an electronic terminal used by the subject). The "goal" may be displayed at the same time or on the same screen as the GA levels and the GMI. The latest or updated goal may be provided to the subject. The subject is thus able to recognize their own latest GA level or the GMI and their own current "goal". Recognition of changes in glycemic biomarkers such as the GA level or the GMI and awareness of the behavior that the subject should implement (also known as "awareness building") may be linked in the conscious or subconscious mind of the subject. This may have a positive impact on behavior change or lifestyle improvement.

### Risk assessment

The term "risk" used in this specification generally refers to the possibility of medical problems arising in connection with glycemic management behavior.

Examples of medical "risks" include, but are not limited to, the onset of illnesses such as hypoglycemia, complications from diabetes, infections, trauma, neoplastic lesions, and mental disorders such as depression, or the possibility of developing such illnesses.

In some embodiments, an assessment regarding whether the subject or the subject's behavior is at risk may be made on the basis of the glucose management index (GMI), the GA levels, the trend of the GA levels, or any combination of the above.

### Embodiment 1

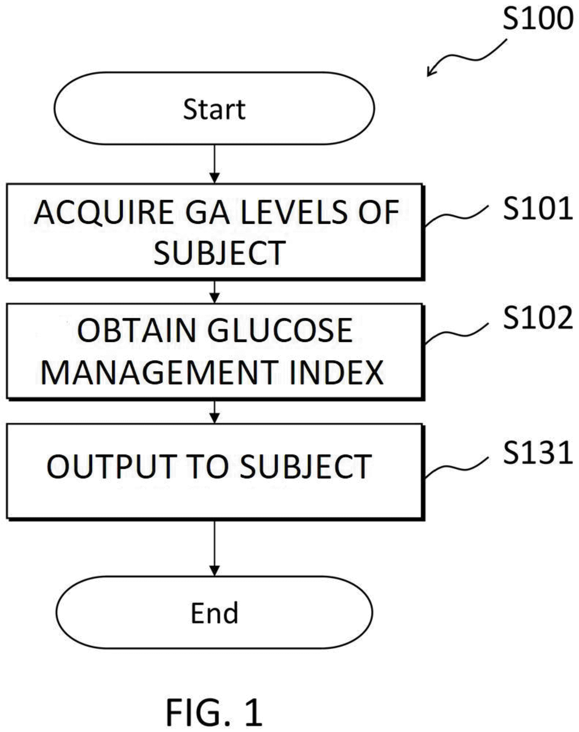

Fig. 1 will be referenced to describe a process S100 of a glucose management method according to an embodiment.

First, GA levels of the subject are acquired (S101). For example, weekly GA levels of the subject are acquired over multiple weeks. To obtain the next GMI, it is necessary to obtain the GA levels at least two points in time. For example, GA[i-1] and GA[i], which are the GA levels at the times t[i-1] and t[i], respectively, are acquired.

Next, the GMI is calculated on the basis of the acquired GA levels (S102). For example, GA[i-1] and GA[i] are used as a basis to calculate the GMI (GMI[i-1, i]) of the period between the times t[i-1] and t[i].

The calculated GMI[i-1, i] is outputted to the subject (S131). By recognizing GMI[i-1, i], the subject can evaluate their own behavior during the period between the times t[i-1] and t[i].

### Embodiment 2

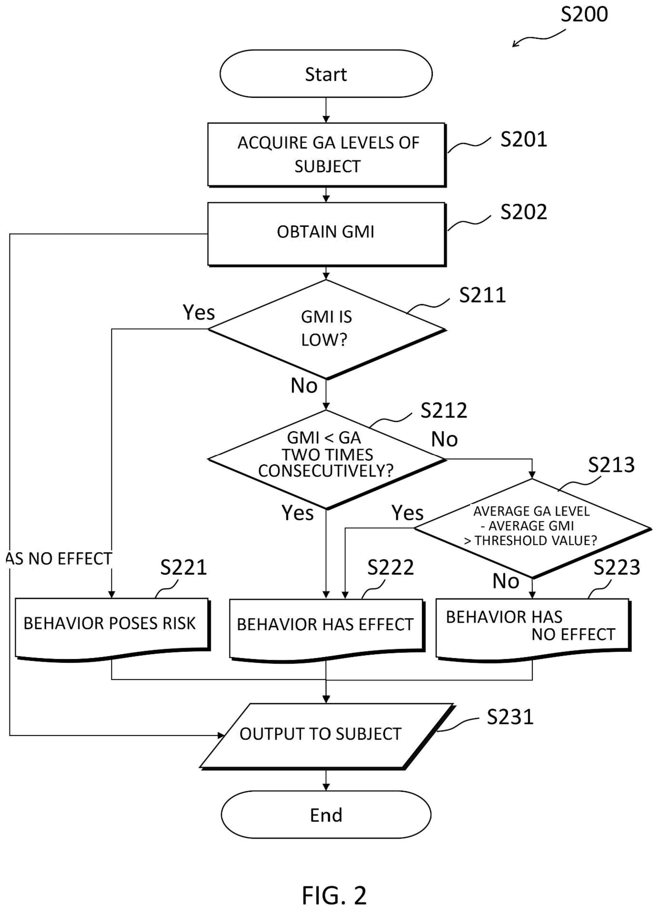

Fig. 2 will be referenced to describe a process S200 of a glucose management method according to an embodiment.

First, GA levels of the subject are acquired (S201). For example, weekly GA levels of the subject are acquired over multiple weeks. To obtain the next GMI, it is necessary to obtain the GA levels at least two points in time. For example, GA[i-1] and GA[i], which are the GA levels at the times t[i-1] and t[i], respectively, are acquired. In the present example, two consecutive weekly GA levels are evaluated in step S212 described later. Accordingly, GA[i-2] is also used in addition to GA[i-1] and GA[i].

Next, the GMI is calculated on the basis of the acquired GA levels (S202). For example, GA[i-1] and GA[i] are used as a basis to calculate GMI[i-1, i] of the period between the times t[i-1] and t[i].

The calculated GMI[i-1, i] is outputted to the subject (S231). By recognizing GMI[i-1, i], the subject can evaluate their own behavior during the period between the times t[i-1] and t[i].

In the present example, a process for interpreting the value and/or trend of the GMI and providing useful comments to the subject on the basis thereof is further included.

If GMI[i-1, i] has fallen below a prescribed threshold value (S211), the subject may be at risk of hypoglycemia for example, and therefore an assessment of "poses risk" is made (S221), and this assessment is outputted to the subject (S231). There is a risk of hypoglycemia in cases of sudden drops in blood glucose levels or prolonged low blood glucose levels. If this assessment is made, a change (easing) of the goal behavior, diagnosis by a doctor, and/or the like may also be proposed, in addition to the GA levels and the GMI, to the subject.

The "poses risk" assessment (S221) may be made on the basis of the GMI alone. For example, the "poses risk" assessment may be made if the GMI falls below 11%. On the other hand, the assessment may also be made by taking into account the GA levels and/or trend of the GA levels, age, medication details, cognitive function, and the like in addition to the GMI. For example, the threshold value for the "poses risk" assessment may be set higher if the subject is at risk for hypoglycemia, such as if the subject is taking medication or if the subject is elderly.

If GMI[i-1, i] has not fallen below the prescribed threshold value (S211), the subject is not at risk of hypoglycemia for example, and therefore an assessment of "no risk" or not "poses risk" is made.

Next, it is assessed whether the GMI continues to show a trend of being lower than the GA level. In the present embodiment, it is assessed whether the GMI is lower than the GA level at least two times consecutively. In other words, the condition is as follows: $GMI \left[i-2, i-1\right] < GA \left[i-1\right] ;$ and $GMI \left[i-1, i\right] < GA \left[i\right] .$

For example, if the GMI is maintained constant at a level lower than the GA level, the GA level will asymptotically approach that value of the GMI. Consequently, the state in which the GMI is lower than the GA level over multiple measurements suggests that the behavior the subject is engaged in is effective in terms of lowering high GA levels.

In Fig. 2, if the GMI is lower than the GA level two times consecutively (S212), it is assessed that the behavior from t[i-1] to t[i] was effective (S222), and this assessment is outputted to the subject (S231).

If the condition that the GMI is lower than the GA level two times consecutively is not met (S212), whether or not the behavior in the most recent period was effective may be further assessed by different criteria.

For example, if the previous GMI was below the GA level but the next GMI exceeded the GA level, it may be assessed that glycemic management shows a worsening trend. As another example, if the previous GMI was above the GA level but the next GMI fell below the GA level, it may be assessed that glycemic management shows an improving trend.

However, in any of the above cases, such an assessment may be avoided, or the assessment itself may be deferred. This is because such assessments may possibly mislead the evaluation of behavior.

In some embodiments, the average of the GA levels and the average of the GMI in two or more periods may be compared. In Fig. 2, when the average of the GMI is sufficiently smaller than the average of the GA levels (average GMI < average GA level - threshold value TH), that is, when the difference therebetween is greater than a certain threshold value (average GA level - average GMI > threshold value TH), it may be assessed that the "behavior has an effect", whereas when this is not the case, it may be assessed that the "behavior has no effect" (S213). In some embodiments, the assessment criterion may be whether the GA levels were showing a downward trend in the period prior to the two periods.

If the GA levels are maintained at a mostly constant level after showing a downward trend (S213, Yes), it is assessed that the most recent "behavior had an effect" (S222). A continual lowering of GA levels is thought to indicate that the subject's behavior during that period was effective. If the GA levels are then maintained mostly constant, the effect of the behavior is thought to be sustained. If the GA levels are displaced slightly while being maintained mostly constant, the GMI is sensitive to the change. Consequently, the effect of the subject's behavior can be evaluated more appropriately by making an assessment in light of the trend of changes in the GA levels, rather than by the GMI alone.

If the GA levels do not show a downward trend in the preceding period (S213, No), it is assessed that the most recent behavior had no effect (S223). For example, if the GA levels were constant in the preceding period, or if the GA levels show an upward trend or at least do not show a downward trend, then it is difficult to say that the GA levels are under control. Accordingly, it cannot be said that the subject's behavior during that period was effective.

In the embodiment illustrated in Fig. 2, the assessments in S211, S212, and S213 are made serially in the diagram, but the order of the assessments is not limited thereto. The assessments may also be made at the same time, in reverse order, or in any order. Similarly, in other diagrams, embodiments, and examples, the order of the steps should not be construed as limiting, but may be changed in any way if such change is reasonable or feasible.

### Embodiment 3

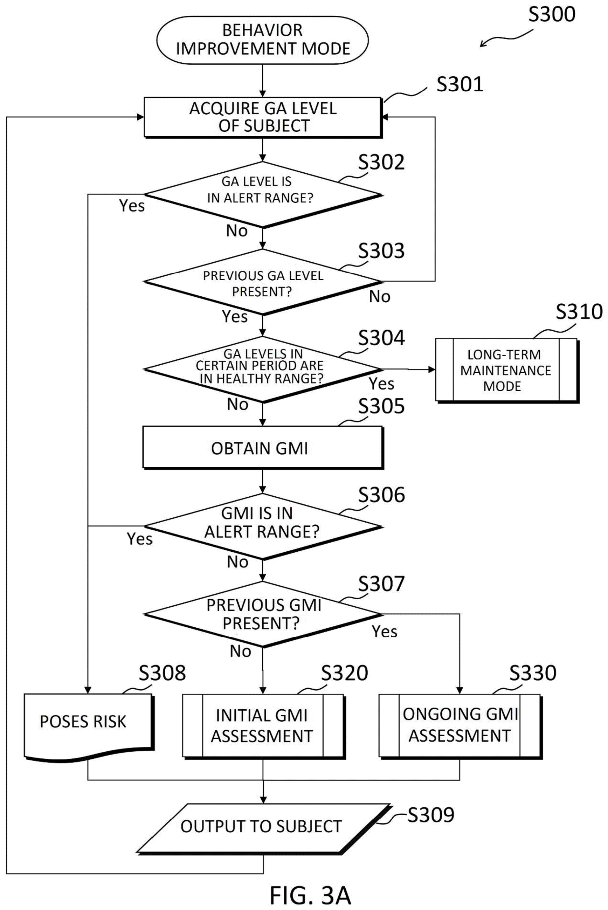

Figs. 3A to 3D will be referenced to describe a process S300 of a glucose management method according to an embodiment.

A basic mode in the present embodiment is the process S300 for encouraging the subject to improve (modify) their behavior (Fig. 3A). First, the GA level of the subject is acquired (S301). If the GA level is extremely low or high (within a numeric range for which an alert is to be issued), it is assessed that the behavior or other circumstances of the subject pose a risk of some kind (S302), and output information indicating "poses risk" (S308) is outputted to the subject (S309). As a non-limiting example, "poses risk" may be assessed when the GA level is below a level such as 11% or 10%, or when the GA level is above a level such as 24%.

If an assessment of no risk is made on the basis of the GA level (S302), it is checked whether the previous GA level, which is necessary to calculate the GMI, is acquired (S303). If the previous GA level is not acquired, that is, if multiple GA levels are not acquired (S303), the next GA level must be acquired (S301).

If multiple GA levels are acquired, these levels are referenced to assess whether the GA levels are in a healthy range (S304). For example, the GA levels over four weeks or one month may be referenced to assess whether all are in a healthy range. If the GA levels are in a healthy range over a period of some extent, there is no need to improve behavior further, and the current good behavior pattern should be continued. In this case, the subject may be supported in a long-term maintenance mode (S310) for continually maintaining the current behavior pattern.

If at least the previous GA level and the latest GA level are acquired, the GMI is calculated on the basis of these GA levels (S305).

If the GMI is extremely low or high (within a numeric range for which an alert is to be issued) (S305), it is assessed that the behavior or other circumstances of the subject pose a risk of some kind (S306), and output information indicating "poses risk" is generated (S308) and outputted to the subject (S309). As a non-limiting example, "poses risk" may be assessed when the GMI is less than a value such as 11% or 10%, or when the GA level is above a level such as 24%.

If an assessment of "no risk" is made on the basis of the GMI (S306), the subject's behavior is evaluated on the basis of the GMI (S320, S330).

For example, the GA levels for only two weeks, or in other words only one GMI, may be acquired in some cases, such as when the present method has just been started or when the present method was not implemented for some time and corresponds to a substantial restart. In such cases, what is called an "initial GMI assessment" process is performed (S320). If the GA levels for three or more weeks are acquired, or in other words, if two or more GMI values are calculated, what is called an "ongoing GMI assessment" process is performed (S330). Each of these processes is described below.

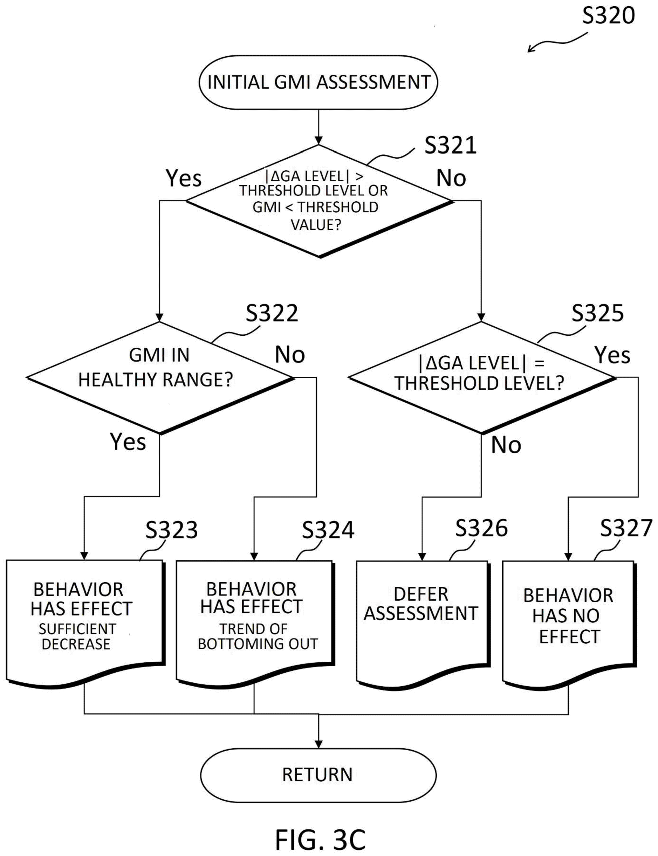

Fig. 3C illustrates a flowchart S320 of the "initial GMI assessment" process according to an embodiment. If the GA level fell sufficiently from the previous GA level or if the initial GMI was sufficiently small (S321), it is assessed that the behavior for the week had an effect (S323, S324). As a non-limiting example, it may be assessed that the GA level fell sufficiently if the change (or decrease) in the GA level is at least 2.5% of the previous GA level and not smaller than a margin of error. As a non-limiting example, it may be assessed that the previous GMI was sufficiently small if the difference between the GMI and the GA level (GMI - GA level) is at least 10% of the previous GA level and not smaller than a margin of error.

In the embodiment illustrated in Fig. 3C, evaluations of the effect of behavior are classified according to the value of the GMI. Specifically, if the GMI is within a healthy range (S322), it is assessed that the GA levels have fallen sufficiently (S323). On the other hand, if the GMI has not yet reached the healthy range (S322), it is assessed that the GA levels are showing a trend of bottoming out (S324).

If the GA levels are showing a trend of bottoming out (S324), a proposal for setting more effective behavior as a goal, a proposal for improving goal behavior, or a proposal for specific goal behavior may be outputted to the subject. Goal behavior related to one or more of diet, exercise, and medication may be improved. In some embodiments, if the subject is unable to improve diet and/or exercise further, the subject may be notified of the need to change medication.

On the other hand, if it is not the case that the GA level fell sufficiently from the previous GA level (the difference "ΔGA level" of the GA level from the previous GA level is greater than a prescribed level) or that the initial GMI was sufficiently small (the initial GMI was less than a prescribed threshold value) (S321), it cannot be said that there was an effect. If there is little or no change in the GA level from the previous GA level (S325), it is assessed that the behavior for the week did not have an effect (S327). If it cannot be said that there was a sufficient change in the GA level from the previous GA level (S321), but there was a slight change (S325), it is not possible to clearly assess whether the behavior had an effect (S323, S324) or did not have an effect (S327). Accordingly, in this case, an assessment is deferred (S326).

When the output information is determined (S323, S324, S326, S327), the output information is outputted to the subject (S319). The GA level for the next week is then acquired (S301). When multiple GMI values are acquired (S307), the "ongoing GMI assessment" process is performed (S330).

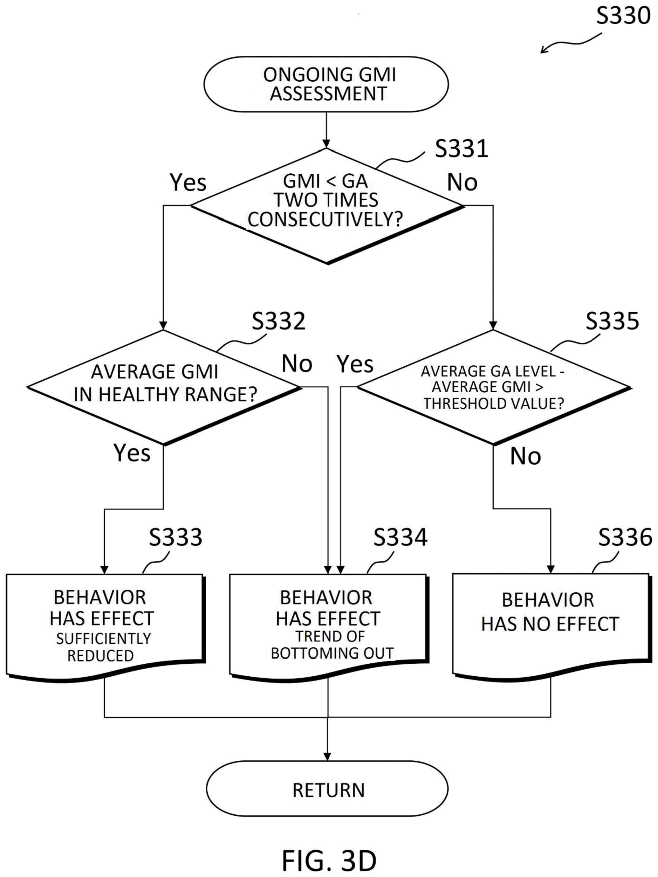

Fig. 3D illustrates a flowchart S330 of the "ongoing GMI assessment" process according to an embodiment. If the GMI is lower than the GA level two times consecutively (S331), it is assessed that the behavior for the week had an effect (S333, S334). In the embodiment illustrated in Fig. 3D, if the average value of the two, or if available, more than two GMI values is within a healthy range (S332), it is assessed that the GA levels have fallen sufficiently (S333). If the average value of the GMI has not yet reached the healthy range (S332), it is assessed that the GA levels are showing a trend of bottoming out (S334).

On the other hand, if the relative magnitudes of the GMI and the GA level have switched places over two weeks (S331), the trend of the GA levels is not clear. If it is simply the case that the GMI and the GA level are close, it cannot be assessed that the GA levels are showing a downward trend. In such a situation, it could be the case that the GA levels are being maintained by effective behavior, or it could be the case that the behavior is not effective and the GA levels are not changing.

In the embodiment in Fig. 3D, if the relative magnitudes of the GMI and the GA level have switched places over two weeks (S331), a further assessment is made on the basis of the average of the GMI and the average of the GA levels (S335). If the average GMI is sufficiently lower than the average GA level (average GMI < average GA level - threshold value TH), it is assessed that the behavior had an effect but the GA levels are showing a trend of bottoming out (S334). Otherwise, it is assessed that the "behavior had no effect" (S336). As a non-limiting example, if the difference between the average GMI and the average GA level (average GA level - average GMI) is greater than a prescribed value (for example, average GA level - average GMI > 1.5%), it may be assessed that the "behavior has an effect".

When the output information is determined (S333, S334, S336), the output information is outputted to the subject (S319). The GA level for the next week is then acquired (S301). If a low GMI continues, the GA levels will also fall accordingly. If this trend continues (S304), the long-term maintenance mode is implemented (S310).

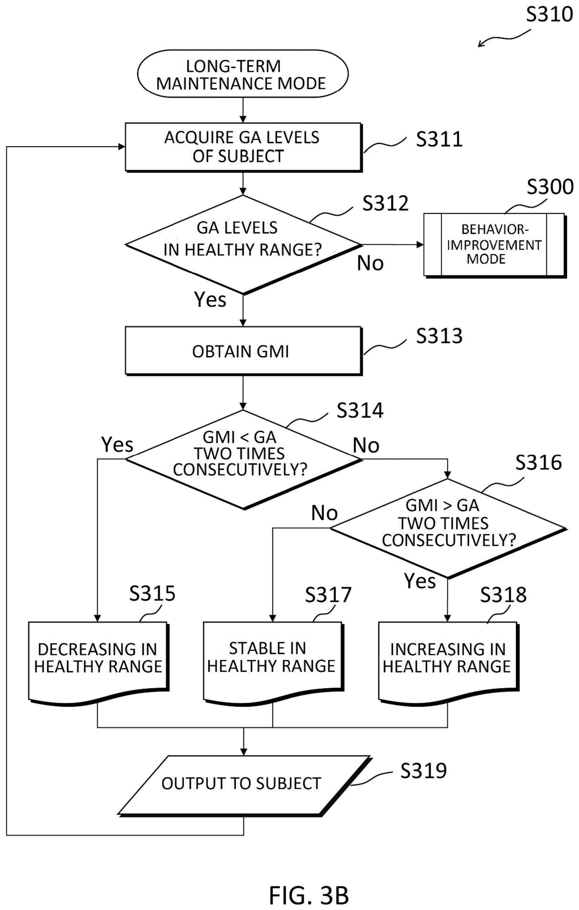

Fig. 3B illustrates a flowchart S310 of the "long-term maintenance mode" according to an embodiment. First, the GA level of the subject is acquired (S311). If the GA level is not in a healthy range (S312), a "behavior improvement mode" is executed immediately (S300). If the GA level is in the healthy range (S312), the long-term maintenance mode is continued. Next, the GMI is calculated (S313).

If the GMI is lower than the GA two times consecutively (S314), it is assessed that the GA levels are in the healthy range and showing a decreasing trend (S315). On the other hand, if the GMI is higher than the GA two times consecutively (S316), it is assessed that the GA levels are in the healthy range and showing an increasing trend (S318). If neither of the above applies (S314, S316), it is assessed that the GA levels are in the healthy range and stable (S317).

The "healthy range" for the GA levels or the GMI may be set on the basis of the subject's intentions, attributes, physiological conditions, medical conditions, external factors, academic theories, general principles, or the like. As a non-limiting example, the "healthy range" for the GA levels or the GMI may be equal to or greater than 11% and less than 16%.

GA levels in the healthy range are proof that the subject successfully achieved effective behavior. However, discontinuing support at this point leads to a deterioration of the GA levels in many subjects. Therefore, the long-term maintenance mode is an effective tool for enabling subjects to maintain ongoing effective behavior, or maintain their motivation to do so.

### Relationship between behavior and GMI

In some embodiments, a relationship or correlation (hereinafter, expressions using either one of these terms shall mean both) between details of behavior that the subject engaged in and/or the subject's goal behavior and the GMI may be obtained. In some embodiments, a relationship between details of behavior and/or goal behavior and the GMI may be obtained for numerous subjects. In some embodiments, a previously obtained relationship between behavior or goal behavior and the GMI and a calculated GMI of a subject may be used as a basis to generate behavior (goal behavior) that the subject should adopt or that is to be proposed to the subject. The goal behavior for a subject may be generated by referring to the GA levels and the trend of the GA levels in addition to the GMI of the subject. An algorithm for generating goal behavior for a subject may be optimized using the GMI or the GA level as evaluation values. For example, an algorithm for generating goal behavior for a subject may be optimized such that the GMI and/or the GA level falls within a range free of risk.

In some embodiments, a relationship between data not derived from GA levels (also referred to as "non-GA-derived data") and the GMI may be obtained. Non-GA-derived data may be classified into data groups such as personal data, health-related data, lifestyle behavior data, and population data, for example, but is not limited thereto. Examples of non-GA-derived data include, but are not limited to, the following:
- Personal data: Personal ID (My Number), age, sex, marital status, number of children, address, type of residence, ownership/non-ownership of automobile, ownership/non-ownership of motorcycle, ownership/non-ownership of bicycle, occupation, job type, job title, annual income, genomic information, religious beliefs, health insurance number, fingerprints, voiceprint, facial recognition information, iris recognition information, time-variable personal thoughts/emotions/desires/wishes, information entered into or used on a smart device, etc.
- Population data: race, nationality, locality, regional area, seasonal factors in region (such as seasonal changes in temperature and humidity), vacation periods (including New Year's, spring break, summer vacation, and/or the like), means of commuting, religion, etc.
- Health-related data: bodyweight, height, body fat percentage, systolic blood pressure, diastolic blood pressure, heart rate, step count, activity level, blood oxygen saturation, body temperature, room temperature, sunshine, UV exposure, a mean blood glucose level, standard deviation, and/or ambulatory glucose profile (AGP) obtained from a continuous glucose monitoring (CGM) or a flash glucose monitoring (FGM) apparatus, health checkup data (which may include HbAlc levels), medical history, prescribed medications, data from a healthcare facility, food or other allergies, medical history and/or health-related data of family members, etc.
- Lifestyle behavior data: dietary habits (including preference for meat, vegetarianism, and/or the like), favorite foods and beverages, meal menus, meal photos, snack photos, exercise habits, wake-up time, bedtime, sleep duration, sleep depth, sleep score, active or passive rest time, eating speed, chewing count/speed/characteristics, food allergies, religious dietary restrictions (including Ramadan and/or the like), presence or absence of exercise, type of exercise, exercise duration, exercise frequency, calories burned through exercise, hobbies, etc.

In some embodiments, a relationship between one or more or minimum heart rate, heart rate variability, body temperature (for example, skin temperature), or other factors and the GMI may be obtained.

### Correlation with CGM

In some embodiments, any parameter of continuous glucose monitoring (CGM) may be estimated on the basis of the GMI. A correlation between a mean blood glucose level calculated from CGM and the GMI may be obtained. Statistical values such as a mean blood glucose level and daily variations such as blood glucose spikes can be acquired from CGM. Correlations between the GMI and these parameters may be obtained. These correlations may be used as a basis to calculate a parameter of the subject from the GMI. This makes it possible to reduce the frequency of CGM use, which requires invasive use over a long period, thus allowing for more comfortable and accurate glycemic management.

### Example of system configuration and operations

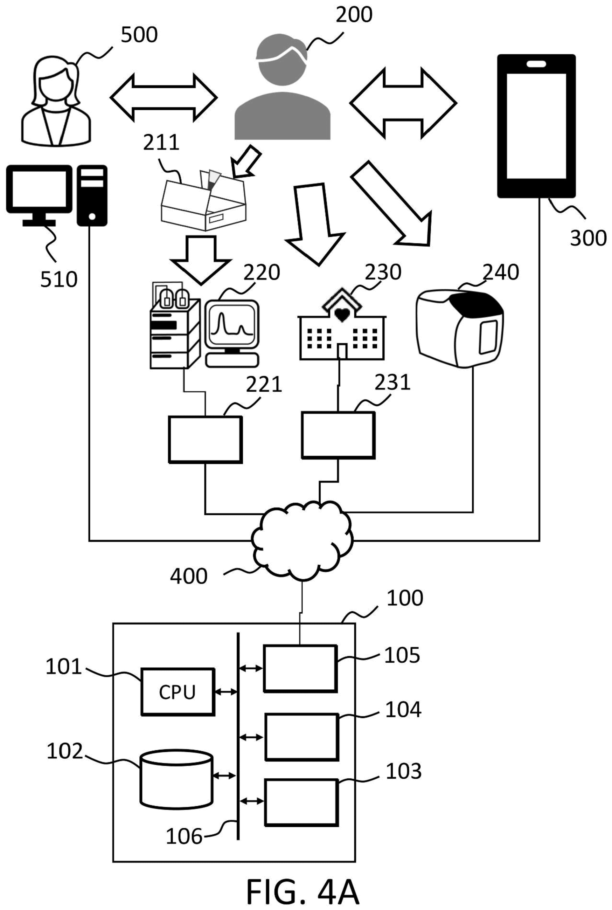

Fig. 4A schematically illustrates a network environment connecting a computational unit 100 and a subject 200 according to an embodiment of the present disclosure.

The computational unit 100 includes a central processing unit (CPU; also referred to as a "processor" and a "computer processor" in this specification) 101, a storage unit (storage) 102, a main memory or memory location 103, a peripheral device 104, and a communication interface 105. The components communicate with the CPU 101 via a communication bus 106 such as a motherboard.

The CPU 101 may be a single-core or multi-core processor, and may also be multiple processors for parallel processing. The CPU 101 may include a computing architecture that processes data signals and implements various architectures or combinations of instruction sets. The CPU has an internal cache (not illustrated), the cache being capable of communicating data at high speed with a registry not illustrated) of the CPU 101. The cache retains data to be used by the CPU 101, which the CPU 101 can process at high speed. Access to the cache is not specified below.

The storage unit or storage 102 is a non-transient storage medium that stores data for providing the functions described in this specification. The storage unit 102 may be a data storage unit (or data repository) for storing data. The storage unit 102 retains programs and data that may be used by the computational unit 100. Examples of the storage unit 102 include, but are not limited to, dynamic random-access memory (DRAM), static random-access memory (SRAM), flash memory, and other kinds of memory devices. Other examples of the storage unit 102 include, but are not limited to, a hard disk, a floppy disk, CD-ROM, DVD-ROM, DVD-RAM, DVD-RW, flash memory, magnetic tape, some other kind of mass storage device for storing information on a persistent basis, and non-volatile memory or similar persistent storage devices and media.

The main memory 103 can store data or instructions that can be executed by the CPU 101. Non-limiting examples include dynamic random-access memory (DRAM), static random-access memory (SRAM), read-only memory (ROM), and flash memory. The CPU 101 can move programs and data retained in the storage unit 102 to the main memory 103 to execute and use the programs and data.

As non-limiting examples, the peripheral device 104 may be other memory, data storage, and/or an electronic display adapter. As a non-limiting example, the communication interface 105 may be a network adapter or the like. The communication interface 105 can communicate with other equipment over a network 400.

There may be multiple ways by which the subject 200 acquires and provides GA levels to the computational unit 100.

In some embodiments, the subject 200 can do mail-in testing. The subject 200 may collect their own body fluid (such as blood and/or saliva) using a prescribed transport kit 211 and send the collected body fluid to a testing center 220. The testing center 220 can use the received body fluid of the subject 200 to measure the GA level of the subject 200. The testing center 220 can communicate with the computational unit 100 over the network 400 via a communication interface 221. The testing center 220 provides the acquired GA level of the subject 200 to the computational unit 100.

In some embodiments, the subject 200 may personally go to a healthcare facility 230. The subject 200 may have their body fluid collected and their GA level measured at the healthcare facility 230. The healthcare facility 230 can use the received body fluid of the subject 200 to measure the GA level of the subject 200. The healthcare facility 230 can communicate with the computational unit 100 over the network 400 via a communication interface 231. The healthcare facility 230 provides the acquired GA level of the subject 200 to the computational unit 100.

In some embodiments, the subject 200 may measure their GA level using a self-monitoring (or at-home) GA measuring apparatus 240. The subject 200 may introduce their body fluid and have their GA level measured by the measuring equipment 240. The measuring equipment 240 can communicate with the computational unit 100 via an internal or externally attached communication interface (not illustrated) and the network 400. The measuring equipment 240 provides the acquired GA level of the subject 200 to the computational unit 100.

The GA level of the subject 200 acquired by one of the ways illustrated or by a way not illustrated is transmitted over the network 400 to the computational unit 100 and received by the communication interface 105, together with additional information (such as an ID of the subject 200, the date and time of body fluid collection, and/or measurement-related information). The information received by the communication interface 105 is temporarily retained in the main memory 103, and saved by being moved to the storage 102 as needed.

The subject 200 possesses a communication-capable electronic terminal 300 (for example, a smartphone, a tablet device, or a personal computer (PC)). The electronic terminal 300 is configured to be connectable to, or to be capable of communicating with, the network 400.

Through the electronic terminal 300, the subject 200 can start, change, stop, or cancel a contract for use of the computational unit 100. The computational unit 100 can establish or carry out communication only with an authorized or contractually valid subject 200 or their electronic device 300, in accordance with personal information, copyright law, civil law, and other laws, and in an ethically and medically appropriate manner. The subject 200 can communicate with the computational unit 100 via the electronic terminal 300 as a legitimate user of the computational unit 100.

The subject 200 may collect their body fluid or have their body fluid collected every week. GA levels are measured therefrom and transmitted to the computational unit 100. The computational unit 100 transmits the received GA level to the electronic terminal 300 of the subject 200. This allows the subject 200 to know their own GA levels via the electronic terminal 300. In a case of using a self-monitoring GA measuring apparatus 240, the subject 200 can know their own GA levels from a display thereof or from the electronic terminal 300 connected to the self-monitoring GA measuring apparatus 240.

By having the subject 200 collect their own body fluid and measure their own GA levels every week, information on GA levels is accumulated in the storage 102.

### Example of functional configuration of system

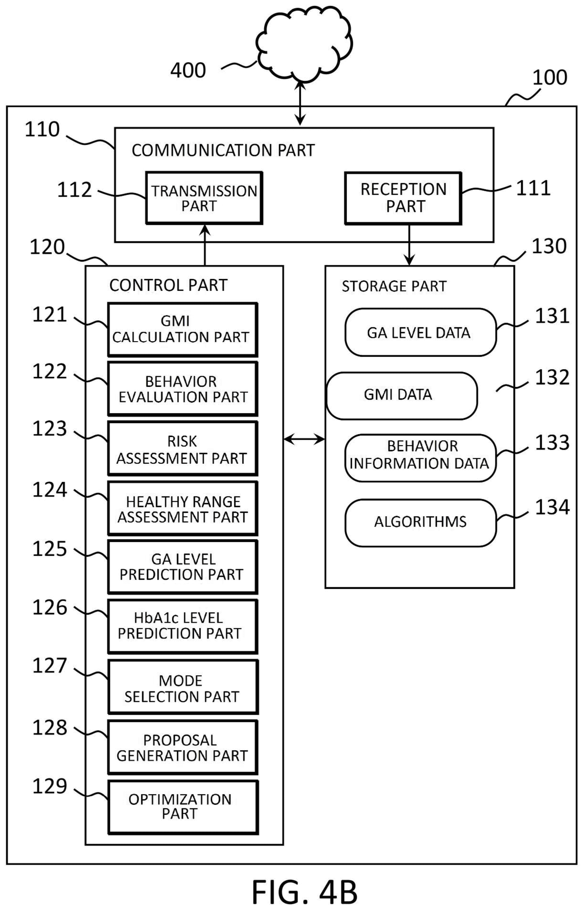

Fig. 4B illustrates components from a functional perspective (functional components) of the computational unit 100 according to an embodiment of the present disclosure. These functional components do not necessarily correspond to physical devices, parts, units, or the like of electronic equipment. Each functional component may be formed from one or more physical devices, parts, or units of electronic equipment, or a portion thereof, or a combination thereof. Expressions referred to as "... part" used in this specification may be replaced with "... means" or "... unit".

The computational unit 100 includes a communication part 110, a control part 120, and a storage part 130.

As a non-limiting example, the communication part 110 may be configured to include a processor for executing programs, a communication I/F, and/or the like. The communication part 110 has a reception part 111 with a function of receiving data from the network 400 and a transmission part 112 with a function of transmitting data to the network 400. The communication part 110 has a function of connecting to the network 400 and communicating with equipment 221, 231, and 240 for measuring or retaining GA levels (also referred to as GA measuring equipment), the user electronic terminal 300, and a counselor terminal 510.

The reception part 111 can receive GA level-related information transmitted from the GA measuring equipment 221, 231, and 240 and a goal, an evaluation, or other input information transmitted from the user terminal 300. The transmission part 112 can transmit output information generated by the control part 120 to a prescribed terminal over the network 400.

As a non-limiting example, the storage part 130 may be configured to have a storage region of a storage medium. The storage part 130 can retain and accumulate data (GA level data) 131 related to GA levels and transmitted from the GA measuring equipment 221, 231, and 240, and calculated GMI data 132. The storage part 130 can retain and accumulate data (goal data) 132 related to a goal of the subject 200 and transmitted from the user electronic terminal 300, information (behavior information data) 133 related the behavior of the subject, and data (evaluation data) 134 related to an evaluation of the output by the subject 200. The storage part 130 stores algorithms to be used by the control part 120.

As a non-limiting example, the control part 120 may be configured to include a processor for executing programs. In some embodiments, the control part 120 may be provided with a GMI calculation part 121. The control part 120 may be further provided with any one, at least one, some, or all of a behavior evaluation part 122, a risk assessment part 123, a healthy range assessment part 124, a GA level prediction part 125, an HbA1c level prediction part 126, a mode selection part 127, and a proposal generation part.

The GMI calculation part 121 calculates the WGMI for the most recent period from the previous GA level and the latest GA level. Alternatively, the GMI calculation part 121 calculates the GMI or the WGMI (hereinafter simply referred to as the "WGMI") from any two GA levels based on the accumulated GA level data. The calculated GMI or WGMI data 132 is accumulated in the storage part 130. The transmission part 112 of the communication part 110 transmits the calculated WGMI. The WGMI is transmitted to the electronic terminal 300 of the subject 200 over the network 400. This allows the subject 200 to know their own WGMI via the electronic terminal 300.

The behavior evaluation part 122 refers to the GMI data 132 to evaluate whether the behavior of the subject 200 had an effect in the period to which the GMI corresponds. For example, if the GMI is lower than the GA level two times consecutively, it may be evaluated that the behavior of the subject 200 had an effect in the period to which the GMI corresponds. If the GA level and GMI switch places from week to week, it is possible that the GA levels have stabilized to some degree. The effect of behavior may be evaluated by referring to statistical behavior over a longer period (such as the average of the GA levels and the average of the GMI, for example). For example, if the average of the GMI is clearly lower than the average of the GA levels (average GMI < average GA - threshold value TH), the GA levels are showing a downward trend. Accordingly, it may be assessed that the behavior of the subject during that period had an effect.

The risk assessment part 123 may refer to the GA levels and/or the GMI to assess whether the subject 200 is or may be at medical risk. For example, the risk assessment part 123 may use the above information as a basis to assess whether the subject 200 is or may be in a state of hypoglycemia. For example, if the GMI is lower than a prescribed value, it may be assessed that the behavior in the corresponding period poses a risk. In general, changes in the GA levels track changes in the GMI. Accordingly, a drop in the GMI can be detected before the GA levels drop to or below a prescribed value to sense at an early stage whether the behavior of the subject 200 poses a risk.

If it is assessed that there is a possibility that the subject 200 is at risk, the computational unit 100 may transmit a message telling the subject 200 that they should receive professional counseling or a diagnosis by a doctor. At the same time or on a separate occasion, the computational unit 100 may also transmit a message stating that the computational unit 100 does not assume any responsibility for the health or illness of the subject 200. The subject 200 can receive the message(s) on the electronic terminal 300 and decide on their own responsibility whether to receive professional counseling or a diagnosis by a doctor.

The healthy range assessment part 124 may refer to the GA level data 131 and/or the GMI data 132 to assess whether the subject 200 is in a healthy range in terms of glycemic management.

The mode selection part 127 refers to any of the GA level data 131, the GMI data 132, and the behavior information 133, or a combination thereof to select or change a mode for supporting the subject 200. For example, if the GA levels and/or the GMI is in a healthy range for a certain period or longer, the CPU 101 may change the support of the subject 200 from a mode for actively encouraging behavior change (behavior change mode) to the long-term maintenance mode. Conversely, for example, if the long-term maintenance mode is being implemented but the GA levels and/or the GMI falls outside a healthy range, the computational unit 100 may initiate implementation of the behavior change mode.

The GA level prediction part 125 predicts future GA levels on the basis of the GMI data 132. For example, if the most recent GMI is maintained, the GA level prediction part 125 may predict the GA level at a certain time in the future. For example, the GA level prediction part 125 may predict the GA level at a certain time in the future on the basis of hypothesized time-series GMI data (change in the GMI over time).

The HbA1c level prediction part 126 may predict future HbA1c levels on the basis of the GMI data 132. For example, if the most recent GMI is maintained, the HbA1c level at a certain time in the future may be predicted. For example, the HbA1c level at a certain time in the future may be predicted on the basis of hypothesized time-series GMI data (change in the GMI over time).

The proposal generation part 128 generates a proposal to the subject 200 on the basis of the GMI data 132, or by additionally referring to other information. The proposal generation part 128 may generate a proposal using a prescribed algorithm 134. For example, the proposal generation part 128 may generate any of the following proposals: the subject 200 should maintain the most recent GMI; the subject 200 should modify their behavior so that the GMI becomes different from the most recent GMI; details of behavior that the subject 200 should engage in; and/or details of behavior that the subject 200 should treat as a goal. When doing so, the proposal generation part 128 may refer to prior GMI data 132 regarding the subject 200, related behavior information data 133 regarding the subject 200, and/or data not regarding the subject 200 (for example, data regarding a person with similar attributes to the subject 200).

The optimization part 129 optimizes algorithms used in glycemic management, such as an algorithm for the behavior evaluation performed by the behavior evaluation part 122, an algorithm for the selection made by the mode selection part 127, and an algorithm used by the proposal generation part 128. For example, the optimization part 129 may tune the relationship between the GMI and behavior on the basis of accumulated GA levels, GMI values, behavior information, and/or the like regarding the subject 200, and optimize an algorithm used to propose behavior that the subject 200 should engage in and/or the like.

In Figs. 4A and 4B, the electronic terminal 300 of the subject 200 is illustrated as a separate unit that can contact the computational unit 100. In some embodiments, the computational unit 100 may be a physical server available via the cloud, for example, the electronic terminal 300 may be a smartphone or a tablet terminal of the subject 200, and both may be configured to be able to contact each other. However, this configuration is an example and should not be interpreted as limiting. In some embodiments, the subject 200 may communicate with the computational unit 100 directly. A physical interface and a unit that performs calculations may be configured in an integrated manner. For example, the computational unit 100 may be configured as part of the electronic terminal 300 of the subject 200. For example, the electronic terminal 300 may be a smartphone, a tablet terminal, a laptop computer, or a desktop computer of the subject 200, or the computational unit 100 may be incorporated into any of the above.

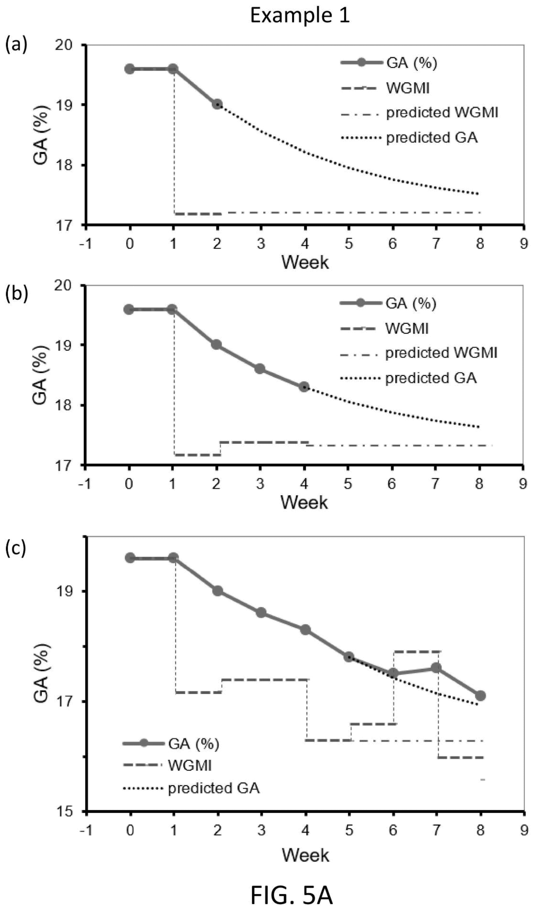

Figs. 5A to 5D will be referenced below to describe an example of glycemic management using the system.

### Example 1

Fig. 5A illustrates the relationship between the GA levels and the WGMI according to an example. The horizontal axis represents weeks, and the vertical axis represents the GA levels and the WGMI. The data shows a hypothetical case based on the actual course of a diabetes patient.

For example, week "1" is the time at which blood was drawn to take the GA level. In the present example, the expression "week n" means, depending on the context, both the time at which blood was drawn and the period from the time "week n-1" to the time "week n".

GA levels were obtained through HPLC analysis of transported fingertip blood obtained by self-puncture. The WGMI, that is, GMI[0, 1], was calculated using Eq. 2 on the basis of GA[0] and GA[1].

As can be seen in Fig. 5A(a), the system referred to the subject's self-reported information and recognized that during the period from week 0 to week 1, "subject consumed one bowl of rice for dinner, ate sweets five times a week, and had habits such as golfing and walking while commuting", conducted an analysis on the basis thereof, and obtained the following result: "daily caloric intake was 2800 kcal, with 52% of calories coming from carbohydrates, and the amount of exercise was approximately 6000 METs per week". At the time of week 1, the GA level was approximately 19.6% (GA[1] = 19.6%).

The system referred to information related to goal behavior entered by the subject and recognized that the goal behavior of the subject at the time of week 1 was set to "start walking and strength training again, cut rice intake by half at every meal, and eat vegetables first". The system referred to the subject's self-reported information and recognized that the subject diligently and actually engaged in the goal behavior every day.

The GA level acquired by the system at the time of week 2 was approximately 19.0% (GA[2] = 19.0%). From GA[1] = 19.6% and GA[2] = 19.0%, the system calculated WGMI[1, 2] = 17.2%. Accordingly, the system predicted the change in GA levels (chain line and dashed line) assuming that the same behavior continued from the time of week 2, or in other words, assuming that the same WGMI was maintained. In this case, the GA levels were predicted to reach approximately 17.5% by week 8.

As can be seen in Fig. 5A(b), the system acquired the weekly GA levels and calculated the WGMI. In week 3, the WGMI rose slightly, but showed the predicted value of 17.4% again in week 4. The system interpreted this to mean that the subject continued to engage in consistent behavior during this period. In fact, the GA levels showed a downward trend from week 1 to week 4, and the GA level for week 3 was substantially the predicted value. Consequently, the system predicted that if the behavior was maintained, the subject would reach the expected GA level in week 8 (chain line and dashed line).

The system referred to information related to goal behavior entered by the subject and recognized that at the time of week 4, the subject changed the goal behavior from the behavior that the subject had been engaged in from week 2 and "increased the number of walking steps from an average of 7000 per day to about 12000, and reduced snacking to approximately one piece of candy once a week". The system referred to the subject's self-reported information and recognized that the subject actually engaged in the goal behavior every day. The system referred to the subject's self-reported information at the time of week 8 and recognized that the subject "consumed a diet with a calorie intake of 2000 kcal, with approximately 45% of calories coming from carbohydrates, and continued to exercise 6400 METs per week".

As can be seen in Fig. 5A(c), the GA level acquired by the system at the time of week 5 was approximately 17.8% (GA[5] = 17.8%). From GA[4] = 18.3% and GA[5] = 17.8%, the system calculated GMI[5, 4] = 16.3%. Accordingly, the system predicted the change in GA levels assuming that the same behavior continued from the time of week 5, or in other words, assuming that the same WGMI was maintained. The system predicted that, in this case, the GA levels would reach approximately 16.9% by week 8. GA[8] = approximately 17.6% assuming continuation of the behavior from week 2 to week 4, clearly demonstrating that increased effort toward the goal behavior contributes to a further decrease in future GA levels.

The GA level acquired by the system at the time of week 6 and the WGMI calculated therefrom were the same as the predicted values again. The GA level acquired by the system in week 7 was 17.6%, higher than the predicted value, and the calculated WGMI rose to 17.9%. The system referred to the subject's self-reported information and recognized that the subject "actually had days when they ate too much and felt that they did not get enough vegetables". The system associated the meal details with the rise in the WGMI, and recorded this relationship in memory. The system acquired the GA level = 17.1% at the time of week 8, and calculated the WGMI = 16.1% on the basis thereof. The system recognized that the subject achieved their goal of reaching the goal GA level of 17.1%, and recorded this information in memory. The system referred to the subject's self-reported information and recognized that "all goals were achieved by reflecting on the lessons from previous weeks". The system associated actual behavior details with the lowering of the WGMI, and recorded this relationship in memory.

In this way, the WGMI was shown to reflect weekly behavior or effort more accurately than the GA levels.

### Example 2

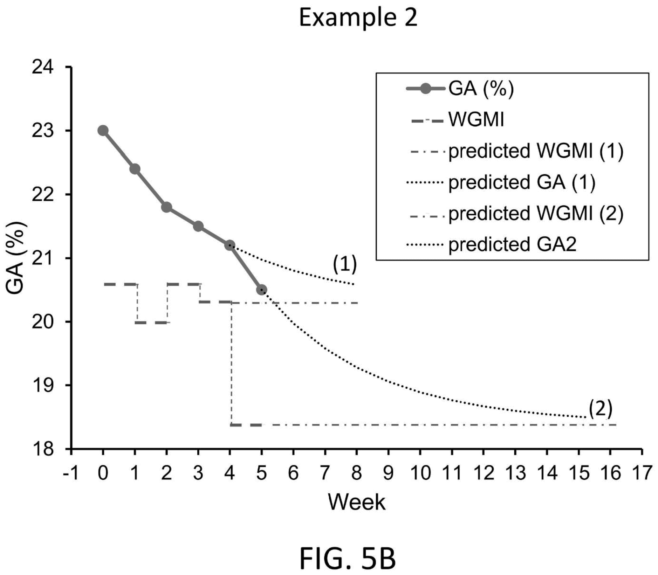

Fig. 5B illustrates the relationship between the GA levels and the WGMI according to an example. The horizontal axis represents weeks, and the vertical axis represents the GA levels and the WGMI. The data shows a hypothetical case based on the actual course of a diabetes patient.

As can be seen in Fig. 5B, the system acquired the GA level at the time of week 4, and calculated the WGMI therefrom. The WGMI was relatively constant between 20.0% and 20.6%, and the GA levels decreased steadily from 23% to 21.2%. Accordingly, the system predicted that if the WGMI was kept constant, the GA level after four would be approximately 20.6% (chain line and dotted line (1)).

At the time of week 5, the system acquired the GA level = 20.5% and calculated the WGMI = 18.4%. On the basis thereof, the system predicted that if the GA levels and the WGMI were kept constant, the GA level after eight weeks would be approximately 18.6% (chain line and dotted line (2)).

The system also acquired the subject's self-reported information at the time of week 5 and recognized that the amount of exercise was increased and dietary intake was limited. The system recognized this as the cause of the steep drop in the WGMI to 18.4%. The system associated the change in behavior with the WGMI and recorded this relationship in memory.

Also, the system assessed that the WGMI = 18.4% and the behavior details did not pose a medical risk, and did not output a "poses risk" alert.

### Example 3

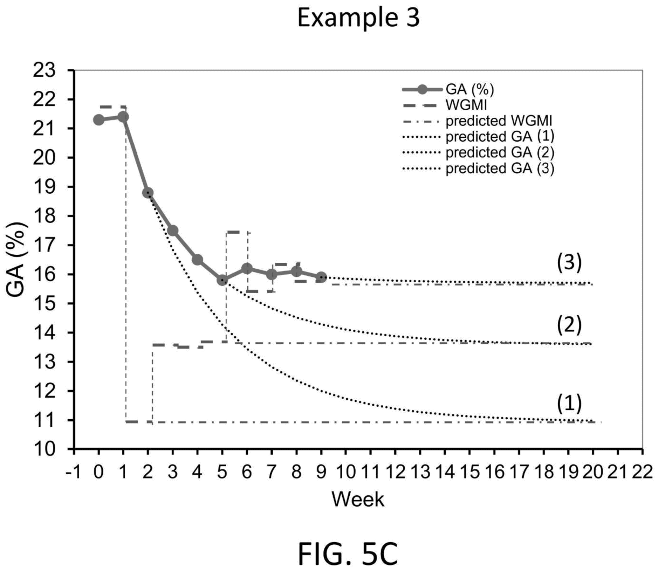

Fig. 5C illustrates the relationship between the GA levels and the WGMI according to an example. The horizontal axis represents weeks, and the vertical axis represents the GA levels and the WGMI. The data shows a hypothetical case based on the actual course of a diabetes patient.

At the time of week 2, the system acquired the GA level and calculated the WGMI. The WGMI was 10.9%, and the GA levels also showed a steep drop from 21.4% to 18.8%. The system predicted that if the WGMI was kept constant, the GA level after eight weeks would be 11.7% (chain line and dotted line (1)).

However, the system assessed that the WGMI 10.9% was below the lower bound of the healthy range and thus posed a risk. Accordingly, the system notified the subject that they should consult a doctor. The system notified the subject's doctor that they should comprehensively review the condition of the subject, and proposed possible adjustments to diet, exercise, and medication. As a result, the doctor determined to reduce the medication dosage, and the system acquired information indicating the same. In accordance therewith, the system included the reduction in medication in the goal behavior of the subject.

By the time of week 5, the GA levels acquired by the system fell to 15.8%, but the calculated WGMI was constant at around 13.6%. On the basis of these values, the system recalculated future GA levels assuming that the WGMI was maintained. As a result, the system predicted that the future GA level would be 13.8% after approximately eight weeks (chain line and dotted line (2)).

The system assessed that the future GA level = 13.8% was appropriate (poses no risk). The system also acquired the subject's self-reported information and recognized that the reduction in medication dosage was also noted in the diary. The system produced output notifying the subject that the effect of the change in medication dosage was apparent, the WGMI was appropriate, and future GA levels were also appropriate. However, the diagnosis by the doctor at this time acknowledged some side effects from the medication, and the doctor decided to reduce the dosage further. The decision by the doctor and the change in medication were entered into the system from a healthcare facility, and the system recorded the details in memory.

The system continued observation up to the time of week 9. The GA levels acquired by the system showed a slight downward trend but were mostly constant between approximately 15.8% and approximately 16.2%. In contrast, the WGMI calculated by the system showed greater variation than the GA levels, varying between approximately 15.3% and approximately 17.4%. In this way, the WGMI tends to fluctuate when the GA levels are mostly constant, or when the WGMI increases or decreases relative to the GA levels. In such cases, rather than referring to individual values of the WGMI, it is better to use the average value or a moving average line to identify the trend of the WGMI.

In the period from week 5 to week 9, the system assessed that the WGMI was in the healthy range, and predicted that if current conditions continued, the GA levels would be maintained at approximately 15.9% (chain line and dotted line (3)). The prediction of future GA level variation was not changed. The system referred to the subject's self-reported information (diary input) and recognized that the medication dosage was constant. On the basis thereof, the system produced output notifying the subject and the doctor that the current status of glycemic management and the glycemic management plan for the past few weeks were all appropriate without any problems.

### Example 4

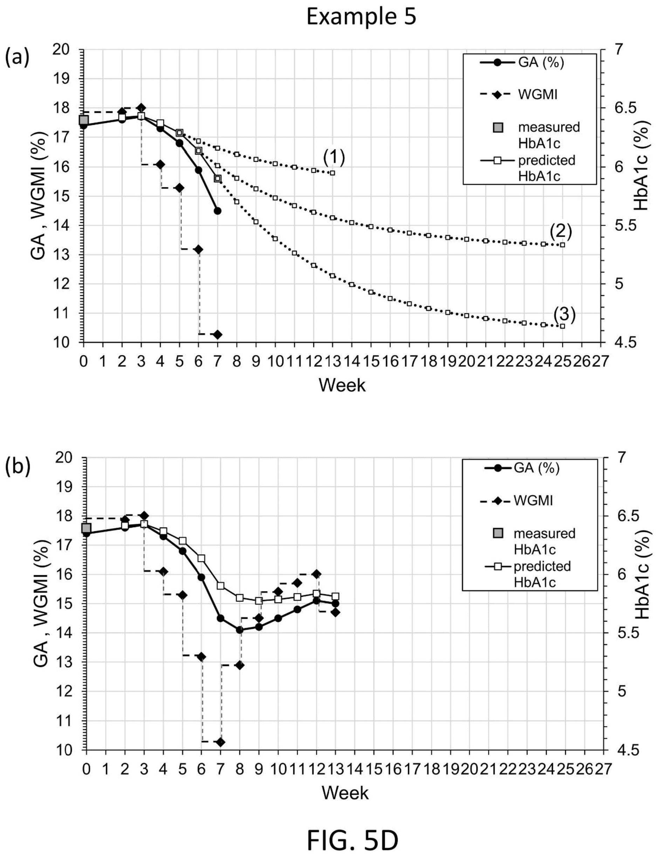

Fig. 5D illustrates the relationship between the GA levels and the WGMI according to an example. The horizontal axis represents weeks, and the vertical axis represents the GA levels and the WGMI. The data shows a hypothetical case based on the actual course of a diabetes patient. Fig. 5D(a) will be referenced to explain the period up to week 7, and Fig. 5D(b) will be referenced to explain the period thereafter up to week 13. Note that although the GA level was not acquired in week 1, the WGMI is correctly calculated with the interval set to two weeks.

As can be seen in Fig. 5D(a), the GA levels acquired by the system were 17.4% in week 0, rose gradually, and were 17.7% in week 3. The calculated WGMI was also high, at 17.9% in week 3. The system outputted these values to the subject. Thereafter, the system referred to the information related to the goal behavior of the subject, recognized that the subject set new goal behavior, and inferred that the subject was making efforts to improve their GA levels. The efforts appeared to be effective, as the WGMI calculated from the GA levels acquired by the system decreased to 16.1% in week 4 and to 15.3% in week 5. The system made an association indicating that the newly set goal behavior and the inferred actual behavior contributed to an improvement in glycemic management, and recorded this relationship in memory. This also demonstrates that the WGMI is sensitive to weekly behavior and more accurately reflects the behavior of the subject.

Accordingly, the system predicted that if the WGMI = 15.3% in week 5 was maintained, the GA level would likely be 15.4% after eight weeks (week 13). The system assessed that the future GA level was not a problem. Furthermore, the system acquired the subject's self-reported information (diary input) and recognized that a behavior change (carbohydrate-restricted diet) occurred during the week of week 5. The system referred to guidelines stating that "sudden carbohydrate-restricted diets are not recommended", and outputted a message recommending that the subject view educational information about proper diet therapy.

The WGMI calculated on the basis of the GA levels acquired by the system fell further to 13.2% in week 6. The system inferred that this was because the subject's behavior changed further. By the time of week 6, the GA levels acquired by the system fell, and the calculated WGMI also fell. The system associated the inference that the behavior changed with the lowering of both the GA levels and the WGMI, and recorded this relationship in memory. Since the GA level acquired by the system was 15.9% and the calculated WGMI was 13.2%, the system assessed that these values were not a problem, and did not output a risk alert. Accordingly, the system predicted that if the WGMI in week 6 was maintained, the GA level would likely be 13.5% after seven weeks (week 13).

The WGMI calculated on the basis of the GA levels acquired by the system fell further to 10.3% in week 7. This was below the threshold value of 11%. For this reason, the system assessed that there was a possibility of a medical problem arising (poses risk), and outputted a risk alert urging the subject to visit a hospital. The system predicted that if the WGMI in week 7 was maintained, future GA levels would likely be less than 11%. The system outputted this prediction to the subject, further outputted additional educational information (video) about diet and exercise therapy, and encouraged the subject to view the information.

As can be seen in Fig. 5D(b), at the time of week 8, the WGMI calculated on the basis of the GA levels acquired by the system was 12.9%, exceeding the threshold value of 11%. Consequently, the system assessed that glycemic management improved. In week 9, the WGMI calculated on the basis of the GA levels acquired by the system rose further to 14.5%. The system assessed that this behavior posed "no risk". In week 10, the WGMI calculated on the basis of the GA levels acquired by the system was 15.5%. The system produced output recommending that the subject continue the current behavior at around the WGMI = 15%. Thereafter, the WGMI calculated on the basis of the GA levels acquired by the system showed a slight upward trend, but was maintained at or below 16%.

In general, standard GA levels are said to fall between 11% and 16%, and thus it is ideal to keep the GA levels below 16%. However, lifestyle patterns and states of health are not completely constant, and the GA levels may exceed the range of standard levels in some cases. Some strategies involve making sudden changes in behavior when the GA levels exceed the standard levels. On the other hand, it is also possible to bring GA levels back down to 16% or below by continuing appropriate lifestyle behavior while monitoring the same using the WGMI. Consequently, even if the GA levels or the GMI temporarily exceeds the range of standard levels or values, it may be recommended to continue the same behavior and continue observation over time.

### Example 5: Prediction of HbA1c levels

As an example, Fig. 5D illustrates actual GA levels of a certain subject from week 0 to week 7, an actual HbA1c level in week 0, the WGMI calculated on the basis of the GA levels, predicted HbA1c levels calculated on the basis of the WGMI, and a prediction of the change in HbA1c levels based on three WGMI patterns. The present example adopts the same GA levels as in example 4.

The HbA1c level acquired by the system was 6.4% in week 0. This level did not exceed 6.5%, the diagnostic threshold for diabetes, but the system assessed that the level was relatively high. The GA levels acquired by the system were 17.4% in week 0, rose gradually, and were 17.7% in week 3. The calculated WGMI was also high, at 17.9% in week 3. The system outputted these values to the subject. Thereafter, the system referred to the information related to the goal behavior of the subject, recognized that the subject set new goal behavior, and inferred that the subject was making efforts to improve their GA levels. The efforts appeared to be effective, as the WGMI calculated from the GA levels acquired by the system decreased to 16.1% in week 4 and to 15.3% in week 5. The system made an association indicating that the newly set goal behavior and the inferred actual behavior contributed to an improvement in glycemic management, and recorded this relationship in memory. In reality, the actual GA levels and the predicted HbA1c levels that were acquired showed a gradual downward trend.

These values also make it clear that the response of GA levels to changes in weekly behavior is slow, and the response of HbA1c levels is even slower. In contrast, the WGMI is sensitive to weekly behavior and more accurately reflects the behavior of the subject.

Accordingly, the system predicted that if the WGMI = 15.1% in week 5 was maintained, the HbA1c level would likely fall below 6 after eight weeks (week 13) (dotted line (1)).

The calculated WGMI fell further to 13.2% in week 6. The system inferred that this was because the subject's behavior changed further. Accordingly, the system predicted that if the WGMI in week 6 was maintained, the HbA1c level would likely fall below 6% and be 5.3% in week 24 (dotted line (2)).

The WGMI calculated from the GA levels acquired by the system fell further to 10.3% in week 7. Accordingly, the system predicted that if the WGMI in week 7 was continued, the HbA1c level would likely be 4.6% in week 24 (dotted line (3)).

In this way, the GMI can be used not only to calculate the current HbA1c level but also to predict future HbA1c levels.

In some embodiments, a prescribed range of HbA1c values (also referred to as the goal HbA1c level range, the range of HbA1c levels as a goal, or the goal range of HbA1c levels) may be set. A range is used interchangeably with an upper bound and/or a lower bound. One or both of an upper bound and a lower bound on the goal HbA1c levels may be set. The goal HbA1c level range may be set as a function of time. For example, a set range of HbA1c levels at a time when the HbA1c level is to be actually tested may be set. Set ranges respectively corresponding to each of a plurality of times may also be set.

In some embodiments, the goal HbA1c level range may be set according to the guidance of a physician or other healthcare professional. In some embodiments, the goal HbA1c level range may be set or proposed by the system. The subject or the healthcare professional may approve the goal HbA1c level range proposed by the system, and the goal HbA1c level range may be set on the basis of the approval. The goal HbA1c level range may be set or proposed on the basis of the user's medical conditions. Examples of the user's medical conditions to be considered include, but are not limited to, age, medical history, disease type (such as type 1 or type 2), and complications. The goal HbA1c level range may be set or proposed by taking these factors into account.

In some embodiments, it may be assessed whether or not a predicted HbA1c level for a certain time based on the GMI is included in the goal HbA1c level range for the same time. A result of the comparison may be outputted. If the predicted HbA1c level is not included in the goal HbA1c level range, a displacement (time-series data) in the GA levels and/or the GMI for causing future HbA1c levels to be included in the goal HbA1c level range may be calculated and outputted. Furthermore, a behavior plan (goal behavior) for bringing about such displacement (time-series data) in the GA levels and/or the GMI may be proposed.

### Example 6: Correlation between CGM and GMI

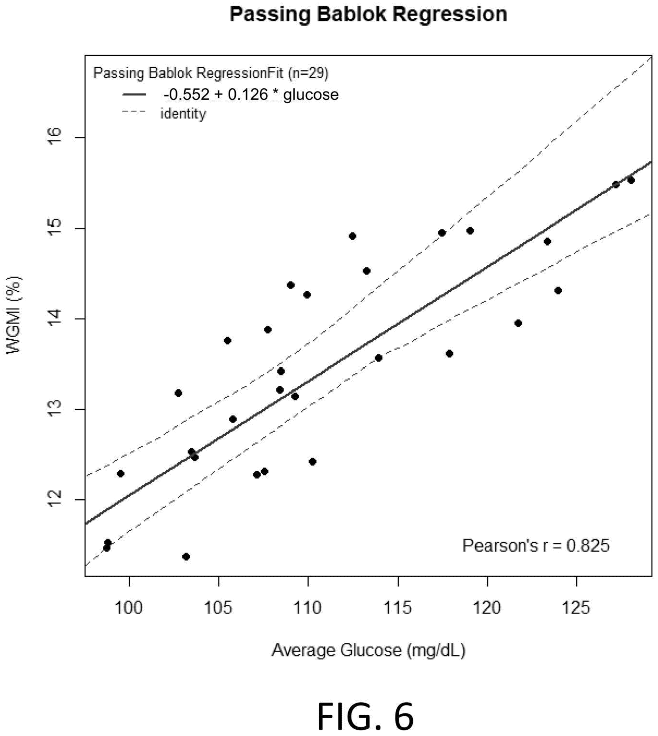

Fig. 6 will be referenced to describe a correlation between CGM and the GMI.

Fig. 6 illustrates a correlation plot of mean glucose levels measured by CGM (referred to as "CGM mean blood glucose levels" in this specification) and the WGMI for five healthy volunteers. These showed a high correlation coefficient of 0.825. The solid line in the plot illustrates a regression line GA or WGMI = 0.126 × glucose value - 0.552 obtained by Passing-Bablok regression, and the dotted lines indicate the 95% confidence interval thereof.

The 95% confidence interval of the regression coefficient illustrated in Fig. 6 had a slope from 0.097 to 0.172 and an intercept from -5.421 to 2.877. The theoretical equation of GA or WGMI = 0.139 × glucose value - 1.49 is included in this range, which demonstrates that the result is consistent with the theoretical equation.

Table 1 indicates correlation coefficients between obtained WGMI and GA levels, durations of indicated hyperglycemia obtained from CGM, and areas of hyperglycemia (time × blood glucose level). It was also observed that the WGMI generally tends to correlate better with various indicators than the GA values.

**Table 1.**

| Pearson's R | WGMI | GA |
|---|---|---|
| Mean glucose (mg/dL) | 0.825 | 0.411 |
| hours above 140 mg/dL | 0.659 | 0.439 |
| hours above 180 mg/dL | 0.394 | 0.353 |
| Area above 140 mg/dL | 0.559 | 0.438 |
| Area above 180 mg/dL | 0.218 | 0.308 |

Consequently, in some embodiments, CGM mean average blood glucose levels may be predicted from the GMI or the WGMI. Alternatively, the GMI or the WGMI may be used as a substitute for some of the numerical values, parameters, statistics, and/or the like obtained by CGM. For example, a correlation between the number of blood glucose spikes seen by CGM and the WGMI may be obtained.

In the present example, future HbAlc levels are predicted on the assumption that the GMI will be constant in the future, but the configuration is not limited thereto. The GMI need not be constant and may change. A formula for converting between GA levels and HbAlc level is known, but this conversion formula is valid when the blood glucose levels or the GA levels are maintained constant over a relatively long period. If the GMI changes from week to week, for example, which is the actual case, such a conversion formula is not valid. The approach according to the present disclosure can show what kinds of HbAlc levels will result from the behavior for the week, in a form that is easy for the subject to understand.

In one embodiment, glycohemoglobin (HbAlc) levels can be predicted with high accuracy using time-series data of glycated albumin (GA) levels. The following describes a configuration of this embodiment in detail.

### 1. Calculation of GA level glucose management index (GMI_{GA})

The following Eq. 8 is used to calculate the glucose management index (GMI_{GA}) from time-series data of GA levels.
[Eq. 8] ${GMI}_{GA} \left[i-1,i\right] = \frac{GA \left[i\right] - GA \left[i-1\right]}{{C}_{GA} \left[i-1,i\right]} + GA \left[i-1\right]$ (Eq. 8)

This equation calculates the glucose management index, which is a dynamic index based on time-series data, by considering the change in GA levels between the times t[i-1] and t[i] and further adding the previous GA level GA[i-1] as a correction term.

The relation between the glucose management index (GMI_{GA}) and mean blood glucose (MBG) can be expressed by the following Eq. 9:
[Eq. 9] $MBG \left[i-1,i\right] = {GMI}_{GA} \left[i-1,i\right] \times {GI}_{Alb}$ (Eq. 9) where GI_{Alb} is the glycation index of albumin. The equation presupposes that the relation between the mean blood glucose MBG and the GA level is such that GA level = GI_{Alb} × MBG, assuming that the glycemic management state is constant and unchanging.

### 2. Calculation of HbAlc level glucose management index (GMI_{HbA1c})

Next, the glucose management index (GMI_{GA}) calculated on the basis of GA levels is used to derive an HbAlc level glucose management index (GMI_{HbA1c}) according to the following Eq. 10. This index dynamically expresses time-series variation of HbAlc and is corrected using the difference between HbAlc levels at different times.
[Eq. 10] ${GMI}_{HbA 1 c} \left[j-1,i\right] = \frac{HbA 1 c \left[j\right] - HbA 1 c \left[j-1\right]}{{C}_{HbA 1 c} \left[j-1,j\right]} + HbA 1 c \left[j-1\right]$ (Eq. 10)

Furthermore, the relation between the HbAlc level glucose management index (GMI_{HbA1c}) and mean blood glucose (MBG) is defined by the following Eq. 11. The clarification of this relation makes possible an integrated understanding of the relationship between dynamic changes in HbAlc levels and blood glucose levels.
[Eq. 11] $MBG \left[j-1,i\right] = {GMI}_{HbA 1 c} \left[j-1,j\right] \times {GI}_{Hb}$ (Eq. 11) where GI_{Hb} is the glycation index of albumin

### 3. Prediction of HbA1c levels

The HbAlc level (HbAlc[q]) at a time t[q] is acquired as a known value. The glucose management index (GMI_{HbA1c}) calculated on the basis of time-series data of GA levels is then used to predict the HbA1c level (HbA1c[r]) at a time t[r] on the basis of the following Eq. 12:
[Eq. 12] $\begin{matrix}HbA 1 c \left[r\right] = HbA 1 c \left[q\right] \\ + \left({GMI}_{HbA 1 c}\left[q, r\right]-HbA1c\left[q\right]\right) \times {C}_{HbA 1 c} \left(t\left[r\right]-t\left[q\right]\right)\end{matrix}$ (Eq. 12)

This equation enables highly accurate prediction of HbAlc levels by combining the HbAlc glucose management index (GMI_{Hba1c}) with time lag and a correction factor C_{HbA1c}.

### 4. Relation between MBG and GMI

The relation between mean blood glucose (MBG) and the GA level glucose management index (GMI_{GA}) is defined by the following Eq. 13:
[Eq. 13] $MBG \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times {GI}_{Alb}$ (Eq. 13)

Also, the relation between mean blood glucose (MBG) and the HbAlc level glucose management index (GMI_{HbA1c}) is defined by the following Eq. 14:
[Eq. 14] $MBG \left[q, r\right] = {GMI}_{HbA 1 c} \left[q, r\right] \times {GI}_{Hb}$ (Eq. 14)

Consequently, the HbA1c glucose management index (GMI_{HbA1c}) can be calculated from Eq. 13 and Eq. 14 as in the following Eq. 15:
[Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15)

Using these equations makes it possible to model an indirect relation present between GA levels and HbAlc levels from the perspective of mean blood glucose.

### 5. Definition of glycation sensitivity ratio (R_{GI}) and method for calculating same

The glycation sensitivity ratio (R_{GI}) is the ratio of glycation susceptibility (GI) to blood glucose levels for different proteins, and is defined by the following Eq. 16. This ratio functions as an important correction coefficient for conversion between GA levels and HbA1c levels.
[Eq. 16] ${R}_{GI} = \frac{{GI}_{Alb}}{{GI}_{Hb}}$ (Eq. 16)

### (1) Case in which HbA1c is temporally stable

If HbA1c levels are relatively stable, the following Eq. 17 and Eq. 18 may be used. In this case, stable R_{GI} calculation is possible because a constant proportional relation exists between GA levels and HbA1c levels.
[Eq. 17] ${GI}_{Alb} \times GA \left[q\right] = {GI}_{Hb} \times HbA 1 c \left[q\right]$ (Eq. 17) [Eq. 18] ${R}_{GI} = \frac{{GI}_{Alb}}{{GI}_{Hb}} = \frac{HbA 1 c \left[q\right]}{GA \left[q\right]}$ (Eq. 18)

This equation can be used if, for example, multiple HbA1c levels are unavailable and only one HbA1c level is acquirable.

### 2) Case in which HbA1c levels vary over time

If HbA1c levels vary over time, making predictions for the specific time t[q] or thereafter requires consideration of the impact of the period prior to the time t[q]. Based on the above, a correction is made according to the following method.

First, if HbA1c levels are acquired at the time t[p] and the subsequent time t[q] (wherein p < q), the mean blood glucose (MBG) in the period from the time t[p] to the time t[q] is defined as in the following Eq. 19:
[Eq. 19] $MBG \left[p, q\right] = {GMI}_{HbA 1 c} \left[p, q\right] \times {GI}_{Hb}$ (Eq. 19) where GMI_{HbA1c}[p, q] represents a glucose management index based on the HbA1c levels from the time t[p] to the time t[q], and GI_{Hb} represents the glycation sensitivity of hemoglobin.

Next, after having acquired GA levels at the times t[p], t[p+1], ..., t[q], MBG is calculated on the basis of the following Eq. 20:
[Eq. 20] $MBG \left[p, q\right] = \frac{{\sum }_{i = p + 1}^{q} \left\{{GMI}_{GA}\left[i-1,i\right]\times \left(t\left[i\right]-t\left[i-1\right]\right)\right\}}{\left(t\left[q\right]-t\left[p\right]\right)} \times {GI}_{Alb}$ (Eq. 20)

In the above, GMI_{GA}[i-1, i] represents the GA level glucose management index, and GI_{Alb} represents the glycation sensitivity of albumin. This equation normalizes changes in GA levels at different times by the time interval, and is designed to average the impact from the time t[p] to the time t[q] in the past.

In addition, the glycation sensitivity ratio (R_{GI}) is calculated according to the following Eq. 21:
[Eq. 21] ${R}_{GI} = \frac{{GI}_{Alb}}{{GI}_{Hb}} = \frac{{GMI}_{HbA 1 c} \left[p, q\right] \times \left(t\left[q\right]-t\left[p\right]\right)}{{\sum }_{k = p + 1}^{q} \left\{{GMI}_{GA}\left[i-1,i\right]\times \left(t\left[i\right]-t\left[i-1\right]\right)\right\}}$ (Eq. 21)

These equations are used to calculate the ratio of the glycation sensitivities of albumin and hemoglobin, and make a correction on the basis of past data spanning from the time t[p] to the time t[q]. This correction further improves the accuracy of predicting HbA1c levels.

Eq. 21 uses two consecutive HbA1c levels, but may use even more HbA1c levels. Doing so improves accuracy even further. In such a case, the glycation sensitivity ratio (R_{GI}) can be calculated according to the following Eq. 22:
[Eq. 22] ${R}_{GI} = \frac{{GI}_{Alb}}{{GI}_{Hb}} = \frac{{\sum }_{k = p + 1}^{q} \left\{{GMI}_{HbA 1 c}\left[k-1,k\right]\times \left(t\left[k\right]-t\left[k-1\right]\right)\right\}}{{\sum }_{k = p + 1}^{q} \left\{{GMI}_{GA}\left[i-1,i\right]\times \left(t\left[i\right]-t\left[i-1\right]\right)\right\}}$ (Eq. 22)

With the above method, the present invention is capable of accurate prediction of HbA1c levels, appropriately taking into account the impact of past data.

According to the present embodiment, there is provided a technology for predicting HbA1c levels with high accuracy using time-series data of GA levels, while significantly reducing the frequency of HbA1c testing. This lessens the burden on both patients and healthcare professionals while also enabling real-time glycemic management.

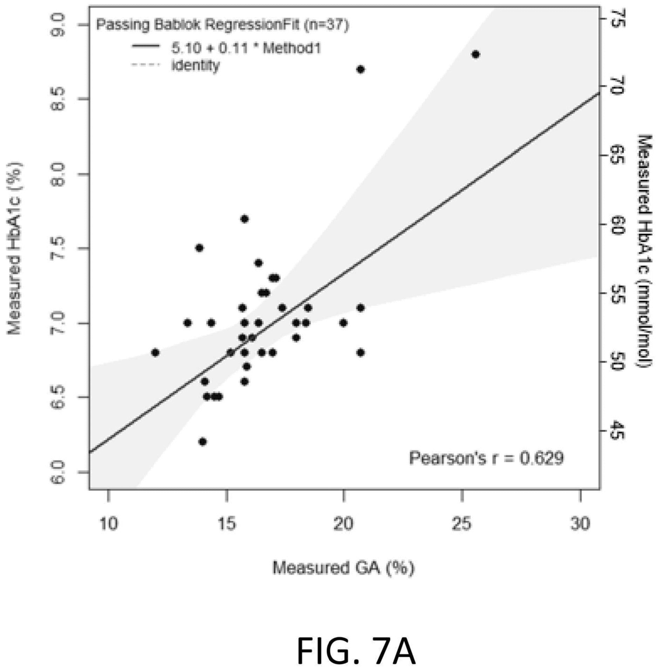

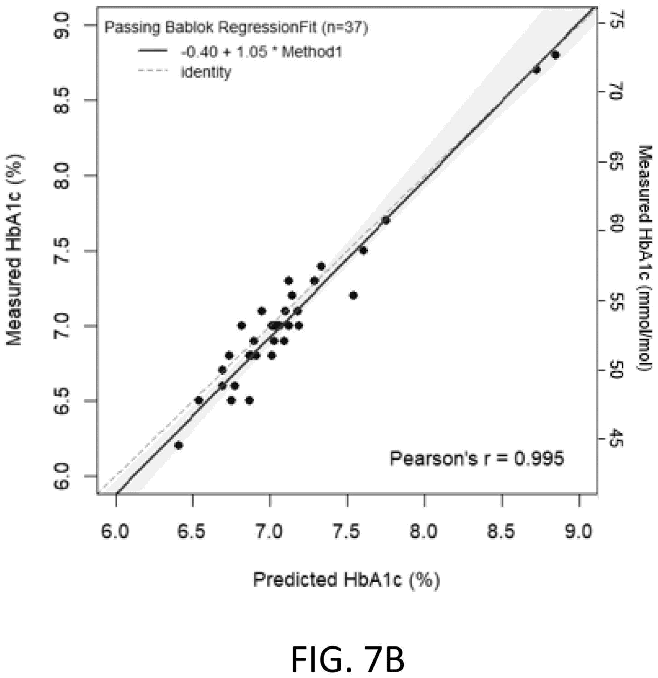

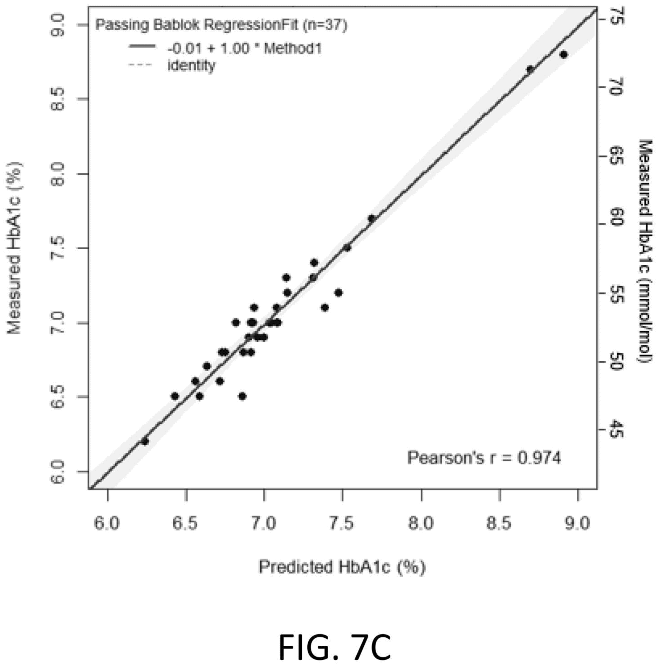

As an example of the present embodiment, Fig. 7 illustrates the results of comparisons between using a conventional method and using the method of the present invention (Eq. 18 and Eq. 21). These comparisons are based on data obtained from the Jinnouchi clinical study (2022-2023, https://doi.org/10.1007%2Fs13300-024-01599-2). In the present example, the participants were patients with type 2 diabetes who visited the hospital three times: an initial visit, an interim visit (five weeks after the initial visit), and a final visit (4 weeks after the interim visit). At each visit, venous blood was drawn and used to measure the HbA1c level. Between the visits, the participants collected fingertip blood at home weekly and mailed the collected blood samples to the hospital, where the blood samples were used for measurement of weekly GA levels. The data indicated in the present example is for 37 out of 44 participants who are patients with type 2 diabetes, minus 7 participants who did not complete at-home measurement during the week of the final visit.

Fig. 7A illustrates the result for the case of predicting HbA1c levels from GA levels using a conventional method. This method involves predicting HbA1c levels from GA levels using linear regression results between the HbA1c level obtained from venous blood drawn at the time of the interim visit (t[p]) and the GA measurement taken at home in the same week.

The Pearson correlation coefficient (Pearson's r) between the predicted and measured levels was 0.629, the slope obtained from the Passing-Bablok regression was 0.11 (95% CI: 0.03 to 0.21), and the intercept obtained from the same was 5.10 (95% CI: 3.55 to 6.39). This result indicates that the predicted levels exhibit significant variation and that there are limitations to the accuracy.

Fig. 7B illustrates the result for the case of applying the method of the present invention and using an approach based on Eq. 18. This method involved calculating the glycation index ratio from the HbA1c level and the GA level measured at the time of the interim visit (t[q]), and predicting the HbA1c level at the time of the final visit (t[r]) using the GA levels in the period from the interim visit (t[q]) to the final visit (t[r]).

The correlation coefficient (Pearson's r) between the predicted and measured levels was extremely high at 0.995, the slope obtained from the Passing-Bablok regression was 1.05 (95% CI: 0.988 to 1.22), and the intercept obtained from the same was -0.40 (95% CI: -1.62 to -0.02). The high correlation coefficient indicates a significant improvement in prediction accuracy compared to the conventional method, but it is observed that the intercept of the linear approximation deviates from zero.

Fig. 7C illustrates the result for the case of further applying an approach based on Eq. 19. This method involved predicting the HbA1c level at the time of the final visit (t[r]) using the GA measurements in the period from the initial visit (t[p]) to the final visit (t[r]) and the HbA1c levels at the time of the initial visit (t[p]) and the interim visit (t[q]).

The Pearson correlation coefficient (Pearson's r) between the predicted and actual levels was 0.975, the slope obtained from the Passing-Bablok regression was 1.00 (95% CI: 0.909 to 1.11), and the intercept obtained from the same was -0.01 (95% CI: -0.81 to 0.63). Moreover, compared to Fig. 7B, Fig. 7C indicates that the intercept of the linear approximation is very close to 0, and it is noteworthy that the intercept almost coincides with the origin (0, 0).

These results confirmed that the method of the present invention significantly improves the accuracy of predicting HbA1c levels compared to the conventional method. In particular, the approach using Eq. 19 is characterized in that the linear approximation of the predicted and measured levels almost coincides with the origin (0, 0), which is excellent in terms of both accuracy and theoretical consistency. This indicates that the method of the present invention has an excellent ability to capture minute variations, making highly reliable glycemic management possible.

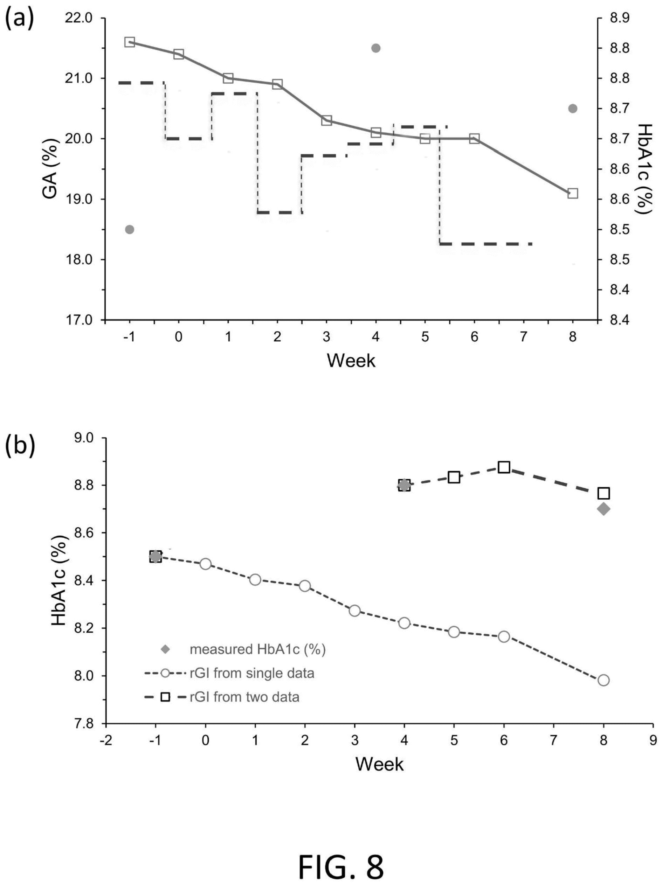

Fig. 8(a) illustrates a graph representing change over time in GA levels, GMI_{GA}, and HbA1c levels actually obtained in the present clinical study. In the graph, □ (square markers with white inside) and the solid line indicate the GA levels, the dashed line indicates GMI_{GA} calculated from the GA levels, and • (round markers with black inside) indicate measured HbA1c levels. Fig. 8(a) illustrates an example indicating different trends in the GA levels and the HbA1c levels. Namely, in this case, the HbA1c levels are increasing even though GA levels are decreasing.

Fig. 8(b) represents a graph illustrating change over time in HbA1c levels predicted by two different methods on the basis of the data in Fig. 8(a), superimposed on the measured HbA1c levels. In the graph, ○ (round markers with white inside) and the thin dashed line indicate the HbA1c levels predicted using Eq. 12 and 15 by using R_{GI} calculated using Eq. 18 from the HbA1c level and the GA level at the time of the initial visit, together with the at-home GA measurements taken thereafter. On the other hand, □ (square markers with white inside) and the thick dashed line indicate the HbA1c levels predicted using Eq. 12 and 15 by using R_{GI} calculated from Eq. 21 using the two HbA1c levels at the time of the initial visit and the interim visit and the at-home GA measurements from week -1 to week 4, together with the at-home GA measurements taken thereafter from week 4.

The difference between these two sets of predicted HbA1c levels typifies the difference in effect between the R_{GI} calculated using Eq. 18 from a single HbA1c level and a single GA level, and the R_{GI} calculated from Eq. 21 using two HbA1c levels and multiple GA measurements. It is thought that if the HbA1c levels are increasing while the GA levels are decreasing, as in the example in Fig. 8(a), the HbA1c levels, which have a longer half-life, more strongly reflect the impact of the increase in blood glucose levels before the start of measurement, while the GA levels more strongly reflect the most recent lowering in blood glucose.

Eq. 18 does not include half-life terms, and the R_{GI} calculation according to the present equation does not reflect such differences in the respective half-lives. Consequently, the HbA1c levels are predicted to decrease in a manner similar to the GA levels. On the other hand, Eq. 21 includes the GMI, which reflects the half-lives. Consequently, the case of using this equation shows that by calculating R_{GI} using two HbA1c levels and multiple GA measurements, the impact of such blood glucose variations in the past can be eliminated to calculate a more accurate glycation sensitivity ratio (see Fig. 8(b)), thus making more accurate HbA1c level prediction possible.

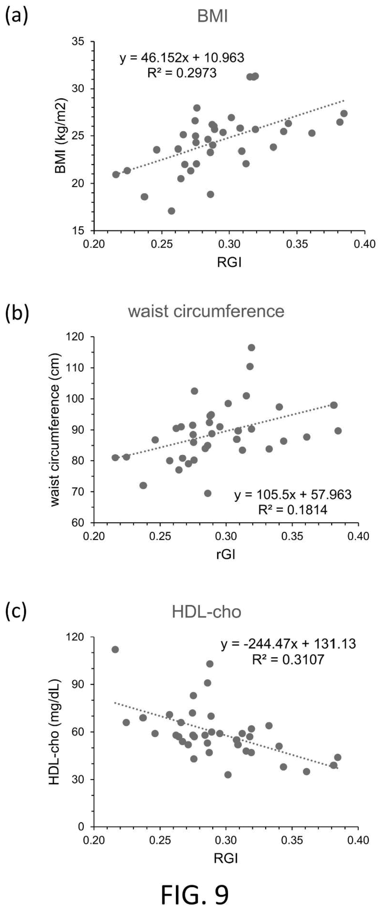

Fig. 9(a) represents a scatter plot illustrating the relation between R_{GI} and BMI for each participant obtained from the present clinical study data. Like Fig. 7(A)(B)(C), the present data is also data for 37 out of 44 participants who are patients with type 2 diabetes, minus 7 participants who did not complete at-home measurement during the week of the final visit. R_{GI} shows a trend of increasing as BMI increases.

Fig. 9(b) represents a scatter plot illustrating the relation between R_{GI} and waist circumference for each participant obtained from the same clinical study data as Fig. 9(a). R_{GI} shows a trend of increasing as waist circumference increases.

These show in particular the importance of calculating R_{GI} by using Eq. 18 or Eq. 21 for users with a high BMI or waist circumference. In other words, these show that the HbA1c levels predicted when using the generally known R_{GI} value of 0.25 diverge from the actual levels more greatly for users with a high BMI or waist circumference. HbA1c levels may be predicted from GA levels measured with the R_{GI} value tentatively assumed to equal 0.25 when actual HbA1c levels are unavailable, such as when blood is not drawn, for example. In this case, a subject with a BMI of 25 or higher or a waist circumference of 85 cm or more may also be notified that the predicted HbA1c levels may have large error or be inaccurate.

Fig. 9(c) illustrates a scatter plot representing the relation between R_{GI} and HDL cholesterol levels for each participant obtained from the same clinical study data as Fig. 9(a). R_{GI} shows a trend of decreasing as HDL cholesterol levels increase.

This shows the possibility of estimating HDL cholesterol levels from GA levels and HbA1c levels. For example, if the R_{GI} value calculated from a GA level and an HbA1c level is greater than a prescribed value (for example, 0.33), the user or a healthcare professional may be notified to check HDL cholesterol levels.

In the embodiments and examples described above, the time constant τ (time constant for decrease of target protein such as albumin or hemoglobin) is set as a general constant. However, in some embodiments, this time constant τ may be set as a parameter specific to the individual. For example, τ may be defined as a parameter of the individual (personalized parameter) using multiple HbA1c levels and GA levels. The personalized time constant τ may be optimized by selectively or combinatorially using parameters from attributes and profile data, measurement data (GA levels, HbA1c levels, CGM data, or combinations thereof), and/or the like for the individual in question. τ may not necessarily be a parameter that changes significantly over a few weeks, but may change over the long term due to changes in an individual's state of health, state of illness, age, and/or the like. Also, τ may be affected by treatment conditions, such as dialysis, for example.

The present disclosure includes the following embodiments:
A001. A method for supporting glycemic management of a subject using a computer system,
   the computer system comprising a processor,
   the method comprising:
      acquiring, by the processor, GA[i] and GA[j], which are glycoalbumin levels (GA levels) of the subject at two times t[i] and t[j] > t[i];
      calculating, by the processor using GA[i] and GA[j] as a basis, a glucose management index (GMI[i, j]) that is a mean blood glucose level equivalent to GA levels in a period between the two times t[i] and t[j]; and
      generating, by the processor, output information based on the glucose management index (GMI[i, j]) and outputting, by the processor, the output information to the subject.
A001b. A method for supporting glycemic management of a subject using a computer system,
   the computer system comprising a processor and a communication unit connected to the processor and capable of communicating with a user interface (for example, a display unit and/or an electronic terminal that can be accessed by the subject),
   the method comprising:
      acquiring, by the processor via the communication unit, GP[i] and GP[j], which are target protein glycation ratios (GP levels) of the subject at two times t[i] and t[j] > t[i];
      calculating, by the processor using GP[i] and GP[j] as a basis, a glucose management index (GMI[i, j]) that is a mean blood glucose level equivalent to GP levels in a period between the two times t[i] and t[j]; and
      generating, by the processor, output information based on the glucose management index (GMI[i, j]) and outputting, by the processor via the communication unit, the output information to the subject.
A002. A method for supporting glycemic management of a subject using a computer system,
   the computer system comprising a processor and a communication unit connected to the processor and capable of communicating with a user interface (for example, a display unit and/or an electronic terminal that can be accessed by the subject),
   the method comprising:
      acquiring, by the processor via the communication unit, GA[i-1] and GA[i], which are GA levels of the subject at two consecutive times t[i-1] and t[i] > t[i-1];
      calculating, by the processor using GA[i-1] and GA[i] as a basis, a glucose management index (GMI[i-1, i]) that is a mean blood glucose level equivalent to GA levels in a period between the two times t[i-1] and t[i]; and
      generating, by the processor, output information based on the glucose management index (GMI[i-1, i]) and outputting, by the processor, the output information to the subject.
A003. The method according to A002 or any embodiment, wherein
   an interval Δt = t[i] - t[i-1] during which to acquire the GA levels is substantially equal to or greater than 1 day and less than or equal to 1 month.
A003b. The method according to A003 or any embodiment, wherein
   the interval Δt = t[i] - t[i-1] during which to acquire the GA levels is substantially equal to or greater than 3 days and less than or equal to 14 days.
A005. The method according to any one of A001 to A003 or any embodiment,
   the computer system further comprising a communication unit connected to the processor and capable of communicating with a user interface (for example, a display unit and/or an electronic terminal that can be accessed by the subject), wherein
   the acquiring GA[i] and GA[j] includes acquiring, by the processor, GA[i] and GA[j] via the communication unit.
A007. The method according to any one of A001 to A005 or any embodiment,
   the computer system further comprising a communication unit connected to the processor and capable of communicating with a user interface (for example, a display unit and/or an electronic terminal that can be accessed by the subject), wherein
   the outputting the output information to the subject includes outputting the output information to the subject via the communication unit.
A011. The method according to A002, A003, or any embodiment, wherein
   the generating the output information includes generating, using the glucose management index (GMI[i-1, i]) as a basis, an evaluation of behavior of the subject during the period, that is, the period from the time t[i-1] to the time t[i].
A012. The method according to A011 or any embodiment, wherein
   the output information includes
   an evaluation indicating that the behavior that the subject engaged in during the period had an effect when the glucose management index (GMI[i-1, i]) is lower than the GA level GA[i].
A013. The method according to A011 or any embodiment, wherein
   the output information includes
   an evaluation indicating that the behavior that the subject engaged in during the period had an effect when
   the glucose management index (GMI[i-1, i]) is lower than the GA level GA[i] two times consecutively, that is, when the following condition is met:
      Condition 1: GMI[i-1, i] < GA[i] and GMI[i-2, i-1] < GA[i-1].
A014. The method according to A013 or any embodiment, wherein
   when condition 1 is not met, the output information includes
   an evaluation indicating that the behavior that the subject engaged in during the period had an effect when
      the average of the glucose management index over at least two times is sufficiently below the average of the GA levels over the at least two times, that is, when the following condition is met:
      Condition 2: average{GMI[i-1, i]} < average{GA[i]} - TH (threshold value) and/or
   when condition 2 is not met, the output information includes
   an evaluation indicating that the behavior that the subject engaged in during the period did not have an effect.
A015. The method according to any one of A012 to A014 or any embodiment, wherein
   the output information includes
   an alert indicating that the behavior that the subject engaged in during the period poses a health risk and/or a notification indicating that goal behavior should be changed when the GMI is below a GMI risk assessment level and/or the GA level is below a GA risk assessment level.
A021. The method according to A001 or any embodiment, further comprising:
   acquiring behavior information related to behavior that the subject engaged in during a period between the times t[i] and t[j]; and
   obtaining a relationship between the behavior information and the glucose management index (GMI[i, j]).
A022. The method according to A021 or any embodiment, wherein
   the behavior information includes self-reported information from the subject and/or biological information acquired in relation to the subject.
A023. The method according to A021 or any embodiment, wherein
   the obtaining the relationship between the behavior information and the glucose management index (GMI[i, j]) includes referencing a database containing behavior information and GA levels regarding a non-subject other than the subject.
A031. The method according to A011 or any embodiment, further comprising:
   generating, using the relationship as a basis, goal behavior that the subject should implement in the future.
A032. The method according to A031 or any embodiment, wherein
   the generating the goal behavior that the subject should implement in the future includes not including in the goal behavior to be generated, or excluding from the goal behavior to be generated, behavior that did not have an effect or that posed a risk in information on behavior that the subject engaged in previously, or lowering a priority of behavior that did not have an effect or that posed a risk.
A035. The method according to A031 or any embodiment, further comprising:
   outputting, to the subject, the generated goal behavior that the subject should implement in the future.
A041. The method according to A031 or any embodiment, wherein
   an algorithm for generating the goal behavior that the subject should implement in the future is optimized using the glucose management index of the subject as an evaluation parameter.
A101. A method for supporting glycemic management of a subject using a computer system,
   the computer system comprising a processor,
   the method comprising:
      (a1) acquiring, by the processor, a first GA level GA[m] of the subject at a first time t[m];
      (b1) acquiring, by the processor, a time-series glucose management index {GMI[i-1, i]} (where i = m+1 to n) from the first time t[m] to a second time t[n]; and
      (c1) calculating, by the processor using the first GA level GA[m] and the time-series glucose management index {GMI[i-1, i]} (where i = m+1 to n) as a basis, the second GA level GA[n] at the second time t[n].
A111. A method for supporting glycemic management of a subject using a computer system,
   the computer system comprising a processor,
   the method comprising:
      (a2) acquiring, by the processor, a first GA level GA[m] of the subject at a first time t[m];
      (b2) acquiring, by the processor, a second GA level GA[n] to serve as a goal at a second time t[n]; and
      (c2) calculating, by the processor using the first GA level GA[m] and the second GA level GA[n] as a basis, a time-series glucose management index {GMI[i-1, i]} (where i = m+1 to n) necessary to reach the second GA level GA[n] at the second time t[n].
A112. The method according to A111 or any embodiment, wherein
   step (c2) includes calculating a time series {GMI[i-1, i]}[k] (where i = m+1 to n) of a plurality of (k, where k ≥ 2) glucose management index values.
A113. The method according to any one of A101 to A112 or any embodiment,
   the computer system further comprising a communication unit connected to the processor and capable of communicating with a user interface (for example, a display unit and/or an electronic terminal that can be accessed by the subject), wherein
   step (a1) and/or step (a2) includes acquiring, by the processor, a first GA level GA[m] of the subject at the first time t[m].
A121. A method for supporting glycemic management of a subject using a computer system,
   the computer system comprising a processor,
   the method comprising:
      (a3) acquiring, by the processor, a first HbA1c level HbA1c[m] of the subject at a first time t[m];
      (b3) acquiring, by the processor, a time-series glucose management index {GMI[i-1, i]} (where i = m+1 to n) from the first time t[m] to a second time t[n];
      (c3) calculating, by the processor using the first HbA1c level HbA1c[m] and the time-series glucose management index {GMI[i-1, i]} (where i = m+1 to n) as a basis, the second HbA1c level HbA1c[n] at the second time t[n];
      (d3) calculating, by the processor, a goal HbA1c level range at the second time t[n];
      (e3) assessing, by the processor, whether or not the second HbA1c level HbA1c[n] is included in the goal HbA1c level range; and
      (f3) outputting, by the processor, a result of the assessment.
A122. The method according to A121 or any embodiment, further comprising:
   (g3) calculating, by the processor, a time-series glucose management index {GMI[i-1, i]} (where i = m+1 to n) such that the second HbA1c level HbA1c[n] at the second time t[n] is included in the goal HbA1c level range.
C001. Software containing computer-executable code for supporting glycemic management of a subject, configured such that the computer-executable code, when executed by the processor,
   causes the processor to:
   acquire GA[i] and GA[j], which are GA levels of the subject at two times t[i] and t[j] > t[i];
   calculate, using GA[i] and GA[j] as a basis, a glucose management index (GMI[i, j]) that is a mean blood glucose level equivalent to GA levels in a period between the two times t[i] and t[j]; and
   generate output information based on the glucose management index (GMI[i, j]) and output the output information to the subject.
C002. The software according to C001 or any embodiment, wherein
   the causing the processor to acquire GA[i] and GA[j] includes causing the processor to acquire GA[i] and GA[j] via a communication unit connected to the processor and capable of communicating with a user interface (for example, a display unit and/or an electronic terminal that can be accessed by the subject).
C011. Software containing computer-executable code for supporting glycemic management of a subject, configured such that
   the computer-executable code, when executed by the processor, causes the processor to execute the method and/or steps according to any one of A001 to A261 or any embodiment. D001. A non-transitory storage medium storing the software according to any of C001 to C201 or any embodiment.
E001. A computer system for supporting glycemic management of a subject, the computer system comprising a processor,

   the processor being programmed to:
   acquire GA[i] and GA[j], which are GA levels of the subject at two times t[i] and t[j] > t[i];
   calculate, using GA[i] and GA[j] as a basis, a glucose management index (GMI[i, j]) that is a mean blood glucose level equivalent to GA levels in a period between the two times t[i] and t[j]; and
   generate output information based on the glucose management index (GMI[i, j]) and output the output information to the subject.
E005. The computer system according to E001 or any embodiment, wherein
   the computer system
      comprises a communication unit connected to the processor and capable of communicating with a user interface (for example, a display unit and/or an electronic terminal that can be accessed by the subject) or
      is configured to be connected to a communication unit capable of communicating with the user interface,
   the causing the processor to acquire GA[i] and GA[j] includes causing the processor to acquire GA[i] and GA[j], which are GA levels of the subject, via the communication unit, and
   the causing the processor to output the output information to the subject includes causing the processor to output the output information to the subject via the communication unit.
E011. A computer system for supporting glycemic management of a subject,
   the computer system comprising a processor,
   the processor being programmed to execute the method and/or steps according to any one of A001 to A201 or any embodiment.
E021. A computer system supporting glycemic control of a subject, the computer system comprising:
   a processor; and
   a non-transitory storage medium storing the software according to any of C001 to C201 or any embodiment.
E022. The computer system according to E021 or any embodiment, further comprising:
   a communication unit capable of communicating with a user interface (for example, a display unit and/or an electronic terminal that can be accessed by the subject).
E101. A computer system supporting glycemic control of a subject, the computer system comprising:
   receiving means for acquiring GA levels of the subject;
   GMI calculating means for calculating a glucose management index on a basis of a plurality of the acquired GA levels; and
   transmitting means for transmitting the glucose management index to a terminal of the subject.
E102. A computer system according to E101 or any embodiment, further comprising one or more of the following:
   behavior evaluating means for evaluating behavior of the subject on a basis of the glucose management index;
   risk assessing means for assessing a risk in behavior of the subject on a basis of the glucose management index and/or the GA levels;
   GA level predicting means for predicting a future GA level on a basis of the glucose management index;
   HbA1c level predicting means for predicting a future HbA1c level on a basis of the glucose management index;
   mode selecting means for selecting, on a basis of the glucose management index and/or the GA levels, a support mode suited to the subject from among a plurality of glycemic management modes;
   proposal generating means for generating, on a basis of the glucose management index, output information including behavior that the subject should adopt or goal behavior; and
   optimizing means for optimizing an algorithm for generating the output information.
B201. A system for supporting glycemic management,
   the system comprising a sensor, a processor, a storage unit, and a user interface, wherein
   the sensor is configured to receive body fluid of a subject, measure a glycoalbumin level (GA level) of the subject, and transmit the measured GA level to the processor,
   the processor is configured to
      receive GA levels at different times (GA[i] and GA[j], where t[j] > t[i]) from the sensor,
      generate a GA glucose management index (GMI_{GA}[i, j]) that is a mean blood glucose level equivalent to GA levels in a period between the two times t[i] and t[j], on a basis of Eq. 1:
         [Eq. 1] ${GMI}_{GA} \left[i, j\right] = \frac{GA \left[j\right] - GA \left[i\right]}{{C}_{GA} \left[i, j\right]} + GA \left[i\right]$ (Eq. 1) where ${C}_{GA} \left[i, j\right] = 1 - exp \left[-\left(t\left[j\right]-t\left[i\right]\right)/{τ}_{Alb}\right] ,$ and
   τ_{Alb} is the time constant for albumin decrease, and
      generate output information based on the GA glucose management index (GMI_{GA}[i, j]) and transmit the output information to the user interface, and
   the user interface is configured to output the output information received from the processor.
B211. The system according to B201 or any embodiment, wherein
   the different times are two consecutive times t[i-1] and t[i] (where t[i] > t[i-1]). B212. The system according to B201, B211, or any embodiment, wherein
   an interval Δt = t[i] - t[i-1] during which to acquire the GA levels is substantially equal to or greater than 3 days and less than or equal to 14 days.
B221. The system according to B201 or any embodiment, further comprising:
   a communication unit connected to the processor and capable of communicating with the sensor and the user interface.
B231. The system according to B201, B211, or any embodiment, wherein
   the output information includes an evaluation of behavior of the subject during a period from the time t[i-1] to the time t[i].
B232. The system according to B231 or any embodiment, wherein
   the output information includes an evaluation indicating that the behavior that the subject engaged in during the period had an effect when the GA glucose management index (GMI_{GA}[i-1, i]) is lower than the GA level GA[i].
B233. The system according to B232 or any embodiment, wherein
   the output information includes
   an evaluation indicating that the behavior that the subject engaged in during the period had an effect when
   the glucose management index (GMI[i-1, i]) is lower than the GA level GA[i] two times consecutively, that is, when the following condition is met:
      Condition 1: GMI[i-1, i] < GA[i] and GMI[i-2, i-1] < GA[i-1].
B234. The system according to B233 or any embodiment, wherein
   when condition 1 is not met, the output information includes
   an evaluation indicating that the behavior that the subject engaged in during the period had an effect when
   the average of the glucose management index over at least two times is sufficiently below the average of the GA levels over the at least two times, that is, when the following condition is met: Condition 2: $average \left\{GMI\left[i-1, i\right]\right\} < average \left\{GA\left[i\right]\right\} - h \left(threshold value\right)$ and/or
   when condition 2 is not met, the output information includes
   an evaluation indicating that the behavior that the subject engaged in during the period did not have an effect.
B235. The system according to any one of B232 to B234 or any embodiment, wherein
   the output information includes
   an alert indicating that the behavior that the subject engaged in during the period poses a health risk and/or a notification indicating that goal behavior should be changed when the GMI is below a GMI risk assessment level and/or the GA level is below a GA risk assessment level.
B241. The system according to B201 or any embodiment, wherein
   the processor is configured to
   acquire behavior information related to behavior that the subject engaged in during a period between the times t[i-1] and t[i], and
   obtain a relationship between the behavior information and the glucose management index (GMI[i-1, i]).
B242. The system according to B241 or any embodiment, wherein
   the behavior information includes self-reported information from the subject and/or biological information acquired in relation to the subject.
B243. The system according to B241 or any embodiment, wherein
   the obtaining the relationship between the behavior information and the glucose management index (GMI[i-1, i]) includes referencing a database containing behavior information and GA levels regarding a non-subject other than the subject.
B244. The system according to B241 or any embodiment, wherein
   the processor is further configured to generate, using the relationship as a basis, goal behavior that the subject should implement in the future.
B245. The system according to B244 or any embodiment, wherein
   the generating the goal behavior that the subject should implement in the future includes not including in the goal behavior to be generated, or excluding from the goal behavior to be generated, behavior that did not have an effect or that posed a risk in information on behavior that the subject engaged in previously, or lowering a priority of behavior that did not have an effect or that posed a risk.
B246. The system according to B244 or any embodiment, wherein
   the processor is further configured to output, to the subject, the generated goal behavior that the subject should implement in the future.
B247. The system according to B244 or any embodiment, wherein
   an algorithm for generating the goal behavior that the subject should implement in the future is stored in the storage unit, and the algorithm is optimized using the glucose management index of the subject as an evaluation parameter.
B251. The system according to B201 or any embodiment, wherein
   the processor is configured to execute the following steps:
   (a1) acquiring, from the sensor, a first GA level GA[m] of the subject at a first time t[m];
   (b1) acquiring, from the sensor, a time-series GA glucose management index {GMI_{GA}[i-1, i]} (where i = m+1 to n) from the first time t[m] to a second time t[n]; and
   (c1) calculating, using the first GA level GA[m] and the time-series glucose management index {GMI_{GA}[i-1, i]} (where i = m+1 to n) as a basis, a second GA level GA[n] at the second time t[n].
B252. The system according to B201 or any embodiment, wherein
   the processor is configured to execute the following steps:
   (a2) acquiring, from the sensor, a first GA level GA[m] of the subject at a first time t[m];
   (b2) acquiring, from the sensor, a second GA level GA[n] to serve as a goal at a second time t[n]; and
   (c2) calculating, using the first GA level GA[m] and the second GA level GA[n] as a basis, a time-series GA glucose management index {GMI_{GA}[i-1, i]} (where i = m+1 to n) necessary to reach the second GA level GA[n] at the second time t[n].
B253. The system according to B252 or any embodiment, wherein
   step (c2) includes calculating a time series {GMI_{GA}[i-1, i]}[k] (where i = m+1 to n) of a plurality of (k, where k ≥ 2) GA glucose management index values.
B261. The system according to B201 or any embodiment, wherein
   the processor is configured to execute the following steps:
   (a) acquiring GA levels of the subject that are a plurality of time-series glycoalbumin levels (GA levels) from a time t[q] to a time t[r] (where q > r);
   (b) calculating, using a time-series glucose management index (GMI_{GA}) of GA levels obtained from the acquired time-series GA levels and a glycation sensitivity ratio R_{GI} as a basis, a GMI (GMI_{HbA1c}[q, r]) of HbA1c levels in a period from t[q] to t[r] using the following Eq. 15 and Eq. 16:
      [Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15) where
      [Eq. 16] ${R}_{GI} = \frac{{GI}_{Alb}}{{GI}_{Hb}}$ (Eq. 16)
   (c) acquiring an HbA1c level (HbA1c[q]) of the subject at the time t[q];
   (d) estimating an HbA1c level (HbA1c[r]) of the subject at the time t[r] (where r > q) from the GMI (GMI_{HBA1c}[q, r]) of the HbA1c levels in the period from t[q] to t[r] and the HbA1c level (HbA1c[q]) of the subject at the time t[q] using the following equation:
      [Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15) and
   (e) generating output information based on the estimated HbA1c level (HbA1c[r]) at the time t[r], and causing the user interface to output the output information.
B262. The system according to B261 or any embodiment, wherein
   R_{GI} is expressed by the following equation:
   [Eq. 18] ${R}_{GI} = \frac{{GI}_{Alb}}{{GI}_{Hb}} = \frac{HbA 1 c \left[q\right]}{GA \left[q\right]}$ (Eq. 18)
B263. The system according to B261 or any embodiment, wherein
   R_{GI} is expressed by the following equation:
   [Eq. 21] ${R}_{GI} = \frac{{GI}_{Alb}}{{GI}_{Hb}} = \frac{{GMI}_{HbA 1 c} \left[p, q\right] \times \left(t\left[q\right]-t\left[p\right]\right)}{{\sum }_{k = p + 1}^{q} \left\{{GMI}_{GA}\left[i-1,i\right]\times \left(t\left[i\right]-t\left[i-1\right]\right)\right\}}$ (Eq. 21)
A201. A method for supporting glycemic management of a subject using a computer system,
   the computer system comprising a sensor, a processor, a storage unit, and a user interface,
   the method comprising:
      measuring, by the sensor, body fluid of a subject to acquire GA levels of the subject;
      transmitting GA levels at different times at different times (GA[i] and GA[j], where t[j] > t[i]) to the processor;
      generating, by the processor, a GA glucose management index (GMI_{GA}[i, j]) that is a mean blood glucose level equivalent to GA levels in a period between the two times t[i] and t[j], on a basis of Eq. 1:
         [Eq. 1] ${GMI}_{GA} \left[i, j\right] = \frac{GA \left[j\right] - GA \left[i\right]}{{C}_{GA} \left[i, j\right]} + GA \left[i\right]$ (Eq. 1) where ${C}_{GA} \left[i, j\right] = 1 - exp \left[-\left(t\left[j\right]-t\left[i\right]\right)/{τ}_{Alb}\right] ,$ and
      τ_{Alb} is the time constant for albumin decrease;
      generating output information based on the GA glucose management index (GMI_{GA}[i, j]) and transmitting the output information to the user interface; and
      outputting, by the user interface, the output information received from the processor to the subject.
A261. The method for supporting glycemic management of a subject using a computer system according to A001 or any embodiment,
   the computer system comprising a sensor, a processor, a storage unit, and a user interface,
   the method comprising:
      (a) measuring, by the sensor, body fluid of a subject to acquire GA levels of the subject that are a plurality of time-series glycoalbumin levels (GA levels) from a time t[q] to a time t[r] (where q > r);
      (b) calculating, by the processor using a time-series glucose management index (GMI_{GA}) of GA levels obtained from the acquired time-series GA levels and a glycation sensitivity ratio R_{GI} as a basis, a GMI (GMI_{HbA1c}[q, r]) of HbA1c levels in a period from t[q] to t[r] using the following equation (Eq. 18):
         [Eq. 18] ${R}_{GI} = \frac{{GI}_{Alb}}{{GI}_{Hb}} = \frac{HbA 1 c \left[q\right]}{GA \left[q\right]}$ (Eq. 18) where
         [Eq. 19] $MBG \left[p, q\right] = {GMI}_{HbA 1 c} \left[p, q\right] \times {GI}_{Hb}$ (Eq. 19)
      (c) acquiring, by the processor, an HbA1c level (HbA1c[q]) of the subject at the time t[q];
      (d) estimating, by the processor, an HbA1c level (HbA1c[r]) of the subject at the time t[r] (where r > q) from the GMI (GMI_{HbA1c}[q, r]) of the HbA1c levels in the period from t[q] to t[r] and the HbA1c level (HbA1c[q]) of the subject at the time t[q] using the following equation:
         [Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15) and
      (d) generating, by the processor, output information based on the estimated HbA1c level (HbA1c[r]) at the time t[r], and transmitting, by the processor, the output information to the user interface;
      (e) outputting, by the user interface, the output information received from the processor to the subject.

Although preferred embodiments of the present invention are illustrated and described herein, it will be clear to a person skilled in the art that such embodiments are provided by way of example only. The present invention is not intended to be limited by the specific examples provided in this specification. Any embodiment or any form disclosed in this specification may independently be combined partially or entirely with other embodiments and forms in any way. For example, one, two, three, or more embodiments can be combined in whole or in part.

When used in this specification, the terms "include" and "comprise" are intended to mean that the configurations and methods include the listed elements, but do not exclude others. Embodiments defined by each of these transitional terms falls within the scope of the present invention. Accordingly, methods, compositions, and configurations can "include" additional steps and components.

A group of items connected by the conjunction "and" should not be read as requiring all of the items to be present in the group, and should be read as "and/or" unless specifically noted otherwise. Similarly, a group of items connected by the conjunction "or" should not be read as requiring mutual exclusivity among items in the group, and should be read as "and/or" unless specifically noted otherwise.

The present invention has been described with reference to the specification above, but the description of the embodiments and the drawings of this specification are not intended to be interpreted in a limiting sense. Numerous variations, changes, and substitutions will occur to a person skilled in the art without departing from the scope of the present invention. Furthermore, it should be understood that all embodiments of the present invention are not limited to the specific depictions, configurations, or relative proportions described herein, which depend on various conditions and variables. It should be understood that various alternatives to the embodiments of the present invention described in this specification may be used to practice the present invention. Consequently, the present invention is considered to encompass such alternative forms, modified forms, variant forms, or their equivalents. The following claims define the scope of the present invention, and it is intended that the methods and structures within these claims, as well as their equivalents, are covered by the claims.

## Claims

1. A system for supporting glycemic management,
the system comprising a sensor, a processor, a storage unit, and a user interface, wherein
the sensor is configured to receive body fluid of a subject, measure a glycoalbumin level (GA level) of the subject, and transmit the measured GA level to the processor,
the processor is configured to
receive GA levels at different times (GA[i] and GA[j], where t[j] > t[i]) from the sensor,
generate a GA glucose management index (GMI_{GA}[i, j]) that is a mean blood glucose level equivalent to GA levels in a period between the two times t[i] and t[j], on a basis of Eq. 1:
[Eq. 1] ${GMI}_{GA} \left[i, j\right] = \frac{GA \left[j\right] - GA \left[i\right]}{{C}_{GA} \left[i, j\right]} + GA \left[i\right]$ (Eq. 1) where ${C}_{GA} \left[i, j\right] = 1 - exp \left[-\left(t\left[j\right]-t\left[i\right]\right)/{τ}_{Alb}\right] ,$ and
τ_{Alb} is the time constant for albumin decrease, and
generate output information based on the GA glucose management index (GMI_{GA}[i, j]) and transmit the output information to the user interface, and
the user interface is configured to output the output information received from the processor.

2. The system according to claim 1, wherein
an interval Δt = t[i] - t[i-1] during which to acquire the GA levels is substantially equal to or greater than 3 days and less than or equal to 14 days.

3. The system according to claim 1, further comprising:
a communication unit connected to the processor and capable of communicating with the sensor and the user interface.

4. The system according to claim 1, wherein
the output information includes an evaluation of behavior of the subject during a period from the time t[i-1] to the time t[i].

5. The system according to claim 3, wherein
the output information includes an evaluation indicating that the behavior that the subject engaged in during the period had an effect when the GA glucose management index (GMI_{GA}[i-1, i]) is lower than the GA level GA[i].

6. The system according to claim 5, wherein
the output information includes
an evaluation indicating that the behavior that the subject engaged in during the period had an effect when
the glucose management index (GMI[i-1, i]) is lower than the GA level GA[i] two times consecutively, that is, when the following condition is met:
Condition 1: GMI[i-1, i] < GA[i] and GMI[i-2, i-1] < GA[i-1].

7. The system according to claim 6, wherein
when condition 1 is not met, the output information includes
an evaluation indicating that the behavior that the subject engaged in during the period had an effect when
the average of the glucose management index over at least two times is sufficiently below the average of the GA levels over the at least two times, that is, when the following condition is met:
Condition 2: average{GMI[i-1, i]} < average{GA[i]} - h (threshold value) and/or
when condition 2 is not met, the output information includes
an evaluation indicating that the behavior that the subject engaged in during the period did not have an effect.

8. The system according to any one of claims 5 to 7, wherein
the output information includes
an alert indicating that the behavior that the subject engaged in during the period poses a health risk and/or a notification indicating that goal behavior should be changed when the GMI is below a GMI risk assessment level and/or the GA level is below a GA risk assessment level.

9. The system according to claim 1, wherein
the processor is configured to
acquire behavior information related to behavior that the subject engaged in during a period between the times t[i-1] and t[i], and
obtain a relationship between the behavior information and the glucose management index (GMI[i-1, i]).

10. The system according to claim 9, wherein
the behavior information includes self-reported information from the subject and/or biological information acquired in relation to the subject.

11. The system according to claim 9, wherein
the obtaining the relationship between the behavior information and the glucose management index (GMI[i-1, i]) includes referencing a database containing behavior information and GA levels regarding a non-subject other than the subject.

12. The system according to claim 9, wherein
the processor is further configured to generate, using the relationship as a basis, goal behavior that the subject should implement in the future, and output, to the subject, the generated goal behavior that the subject should implement in the future.

13. The system according to claim 12, wherein
the generating the goal behavior that the subject should implement in the future includes not including in the goal behavior to be generated, or excluding from the goal behavior to be generated, behavior that did not have an effect or that posed a risk in information on behavior that the subject engaged in previously, or lowering a priority of behavior that did not have an effect or that posed a risk.

14. The system according to claim 12, wherein
an algorithm for generating the goal behavior that the subject should implement in the future is stored in the storage unit, and the algorithm is optimized using the glucose management index of the subject as an evaluation parameter.

15. The system according to claim 1, wherein
the processor is configured to execute the following steps:
(a1) acquiring, from the sensor, a first GA level GA[m] of the subject at a first time t[m];
(b1) acquiring, from the sensor, a time-series GA glucose management index {GMI_{GA}[i-1, i]} (where i = m+1 to n) from the first time t[m] to a second time t[n]; and
(c1) calculating, using the first GA level GA[m] and the time-series glucose management index {GMI_{GA}[i-1, i]} (where i = m+1 to n) as a basis, a second GA level GA[n] at the second time t[n].

16. The system according to claim 1, wherein
the processor is configured to execute the following steps:
(a2) acquiring, from the sensor, a first GA level GA[m] of the subject at a first time t[m];
(b2) acquiring, from the sensor, a second GA level GA[n] to serve as a goal at a second time t[n]; and
(c2) calculating, using the first GA level GA[m] and the second GA level GA[n] as a basis, a time-series GA glucose management index {GMI_{GA}[i-1, i]} (where i = m+1 to n) necessary to reach the second GA level GA[n] at the second time t[n].

17. The system according to claim 1, wherein
the processor is configured to execute the following steps:
(a) acquiring GA levels of the subject that are a plurality of time-series glycoalbumin levels (GA levels) from a time t[q] to a time t[r] (where q > r);
(b) calculating, using a time-series glucose management index (GMI_{GA}) of GA levels obtained from the acquired time-series GA levels and a glycation sensitivity ratio R_{GI} as a basis, a GMI (GMI_{HbA1c}[q, r]) of HbA1c levels in a period from t[q] to t[r] using the following Eq. 15 and Eq. 16:
[Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15) where
[Eq. 16] ${R}_{GI} = \frac{{GI}_{Alb}}{{GI}_{Hb}}$ (Eq. 16)
(c) acquiring an HbA1c level (HbA1c[q]) of the subject at the time t[q];
(d) estimating an HbA1c level (HbA1c[r]) of the subject at the time t[r] (where r > q) from the GMI (GM_{HbA1c}[q, r]) of the HbA1c levels in the period from t[q] to t[r] and the HbA1c level (HbA1c[q]) of the subject at the time t[q] using the following equation:
[Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15) and
(e) generating output information based on the estimated HbA1c level (HbA1c[r]) at the time t[r], and causing the user interface to output the output information.

18. The system according to claim 17, wherein
R_{GI} is expressed by the following equation:
[Eq. 18] ${R}_{GI} = \frac{{GI}_{Alb}}{{GI}_{Hb}} = \frac{HbA 1 c \left[q\right]}{GA \left[q\right]}$ (Eq. 18)

19. The system according to claim 17, wherein
R_{GI} is expressed by the following equation:
[Eq. 21] ${R}_{GI} = \frac{{GI}_{Alb}}{{GI}_{Hb}} = \frac{{GMI}_{HbA 1 c} \left[p, q\right] \times \left(t\left[q\right]-t\left[p\right]\right)}{{\sum }_{k = p + 1}^{q} \left\{{GMI}_{GA}\left[i-1,i\right]\times \left(t\left[i\right]-t\left[i-1\right]\right)\right\}}$ (Eq. 21)

20. A method for supporting glycemic management of a subject using a computer system,
the computer system comprising a sensor, a processor, a storage unit, and a user interface,
the method comprising:
measuring, by the sensor, body fluid of a subject to acquire GA levels of the subject;
transmitting GA levels at different times at different times (GA[i] and GA[j], where t[j] > t[i]) to the processor;
generating, by the processor, a GA glucose management index (GMI_{GA}[i, j]) that is a mean blood glucose level equivalent to GA levels in a period between the two times t[i] and t[j], on a basis of Eq. 1:
[Eq. 1] ${GMI}_{GA} \left[i, j\right] = \frac{GA \left[j\right] - GA \left[i\right]}{{C}_{GA} \left[i, j\right]} + GA \left[i\right]$ (Eq. 1) where ${C}_{GA} \left[i, j\right] = 1 - exp \left[-\left(t\left[j\right]-t\left[i\right]\right)/{τ}_{Alb}\right] ,$ and
τ_{Alb} is the time constant for albumin decrease;
generating output information based on the GA glucose management index (GMI_{GA}[i, j]) and transmitting the output information to the user interface; and
outputting, by the user interface, the output information received from the processor to the subject.

21. The method for supporting glycemic management of a subject using a computer system according to claim 20,
the method comprising:
(a) measuring, by the sensor, body fluid of a subject to acquire GA levels of the subject that are a plurality of time-series glycoalbumin levels (GA levels) from a time t[q] to a time t[r] (where q > r);
(b) calculating, by the processor using a time-series glucose management index (GMI_{GA}) of GA levels obtained from the acquired time-series GA levels and a glycation sensitivity ratio R_{GI} as a basis, a GMI (GMI_{HbA1c}[q, r]) of HbA1c levels in a period from t[q] to t[r] using the following equation (Eq. 18):
[Eq. 18] ${R}_{GI} = \frac{{GI}_{Alb}}{{GI}_{Hb}} = \frac{HbA 1 c \left[q\right]}{GA \left[q\right]}$ (Eq. 18) where
[Eq. 19] $MBG \left[p, q\right] = {GMI}_{HbA 1 c} \left[p, q\right] \times {GI}_{Hb}$ (Eq. 19)
(c) acquiring, by the processor, an HbA1c level (HbA1c[q]) of the subject at the time t[q];
(d) estimating, by the processor, an HbA1c level (HbA1c[r]) of the subject at the time t[r] (where r > q) from the GMI (GMI_{HbA1c}[q, r]) of the HbA1c levels in the period from t[q] to t[r] and the HbA1c level (HbA1c[q]) of the subject at the time t[q] using the following equation:
[Eq. 15] $\begin{matrix}{GMI}_{HbA 1 c} \left[q, r\right] = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right)} \times \frac{{GI}_{Hb}}{{GI}_{Alb}} \\ = \frac{{\sum }_{i = q + 1}^{r} \left\{{GMI}_{GA}\left[i-1,i\right]\times \left(t\left[i\right]-t\left[i-1\right]\right)\right\}}{\left(t\left[r\right]-t\left[q\right]\right) \times {R}_{GI}}\end{matrix}$ (Eq. 15) and
(d) generating, by the processor, output information based on the estimated HbA1c level (HbA1c[r]) at the time t[r], and transmitting, by the processor, the output information to the user interface;
(e) outputting, by the user interface, the output information received from the processor to the subject.
